# EUROPÄISCHE PATENTSCHRIFT

(11) **EP 3 973 971 B1**
(45) Veröffentlichungstag und Bekanntmachung des Hinweises auf die Patenterteilung: **31.12.2025**
(21) Anmeldenummer: 20209988.3
(22) Anmeldetag: 26.11.2020
(51) Int. Cl.: A61K 35/74, A61P 17/10

(54) **PROBIOTISCHE PHARMAZEUTISCHE ZUSAMMENSETZUNG**
PHARMACEUTICAL COMPOSITIONS COMPRISING A PROBIOTIC
COMPOSITIONS PHARMACEUTIQUE COMPRENANT UN PROBIOTIQUE

(30) Priorität: 28.09.2020 EP 20198694
(43) Veröffentlichungstag der Anmeldung: 30.03.2022
(73) Patentinhaber: INSTITUT ALLERGOSAN Holding GmbH, 8055 Graz (AT)
(72) Erfinder: AMMER, Richard, 58644 Iserlohn (DE)
(74) Vertreter: Schwarz & Partner Patentanwälte GmbH

(56) Entgegenhaltungen:
- EP-A1- 2 228 067
- EP-A1- 3 176 175
- EP-B1- 3 484 585
- WO-A1-2016/149687
- WO-A1-2019/180748
- WO-A1-2020/120670
- WO-A1-2020/234867
- US-B2- 8 737 783
- DATABASE WPI Week 202006, Derwent World Patents Index; AN 2020-67543U, XP002802702
- DATABASE WPI Week 202009, Derwent World Patents Index; AN 2020-B3096W, XP002802703
- DATABASE WPI Week 202007, Derwent World Patents Index; AN 2020-84903Y, XP002802704
- DATABASE WPI Week 201615, Derwent World Patents Index; AN 2015-626854, XP002802705

## Beschreibung

Die Erfindung betrifft eine pharmazeutische Zusammensetzung, ein Wasserbad mit der pharmazeutischen Zusammensetzung und ein Verfahren zur Herstellung des Wasserbads.

### Hintergrund der Erfindung

Sowohl das intestinale als auch das dermale Mikrobiom sind für die menschliche Gesundheit von Bedeutung. Das intestinale Mikrobiom beeinflusst zahlreiche Immunfunktionen und ist wahrscheinlich auch in die Entwicklung atopischer Erkrankungen involviert. Daneben sind aber auch Interaktionen zwischen dermalen Mikroorganismen und dem spezifischen Immunsystem bekannt. In den vergangenen Jahren sind die Effekte von Pro- und Präbiotika ausführlich untersucht worden. Es hat sich gezeigt, dass sowohl die interne als auch die externe Anwendung pro- oder präbiotischer Präparate bei der Behandlung atopischer Erkrankungen wirksam sein könnte.

Neurodermitis (atopische Dermatitis, AD) ist eine chronische oder chronisch-rezidivierende, nicht ansteckende Hauterkrankung, deren klassische Morphologie und Lokalisation altersabhängig unterschiedlich ausgeprägt ist und die meist mit starkem Juckreiz einhergeht. Der Krankheitsverlauf ist wechselhaft und die Symptome treten häufig in Schüben auf, die in Dauer und Schwere unterschiedlich stark ausgeprägt sein können. Sie sind gekennzeichnet durch trockene Haut mit geröteten, entzündeten Stellen (Ekzeme), flächenhafte Verdickungen und Vergröberung der Haut (Lichenifikation) sowie Knötchen und Pusteln (Prurigo-Knoten).

Im Säuglingsalter treten die Ekzeme streckseitig an Armen und Beinen, im Gesicht sowie am Kopf auf. Bei älteren Kindern und Erwachsenen finden sich häufig Beugenekzeme in den Ellenbeugen oder in den Kniekehlen. In Abhängigkeit von hautbelastenden Tätigkeiten können auch Handekzeme auftreten.

Gemäß versorgungsepidemiologischen Analysen aus Deutschland nehmen rund 23% der Säuglinge und Kleinkinder, 8% der Schulkinder und 2-4% der Erwachsenen Gesundheitsleistungen aufgrund der Neurodermitis in Anspruch.

Die AD ist eine komplexe Erkrankung und die Ursachen können vielfältig sein. Unterschiedliche genetische Faktoren kommen als prädisponierende Faktoren in Frage, dabei spielen besonders Veränderungen von Genen eine Rolle, die mit der Barrierefunktion der Haut assoziiert sind, wie beispielsweise loss of function-Mutationen des Filaggrin-Gens. Das Genprodukt Filaggrin spielt eine wichtige Rolle bei der Transformation von Keratinozyten zu Korneozyten. Spaltungsprodukte des Fillagrins sorgen zudem für die Wasserbindung und die Aufrechterhaltung des niedrigen pH-Wertes des Stratum corneums.

Weiterhin werden Umwelteinflüsse diskutiert, die sich aus dem Leben in Industriestaaten, insbesondere in urbanisierten Gegenden, ergeben, dazu gehört der Einfluss von Luftverschmutzung wie z.B. durch Autoabgase, aber auch das vorwiegende Leben in geschlossenen und geheizten Räumen, die geringere mikrobielle Exposition und eine übertriebene Verwendung von Seifen und reinigenden Kosmetika.

Im Zusammenspiel der unterschiedlichen Faktoren kommt es zu einer Beeinträchtigung der Barrierefunktion der Haut sowie zu einer Dysregulation des Immunsystems, wobei nicht eindeutig klar ist, wie sich beide Effekte gegenseitig bedingen.

Ein Krankheitsschub wird vermutlich dadurch ausgelöst, dass antigenpräsentierende Zellen (Langerhans-Zellen) Antigene an ihrer Zellmembran binden und diese dann über MHC-Klasse 2 Rezeptoren CD4+ T-Zellen präsentieren. Die aktivierten APCs (antigen-presenting cells) sezernieren Interleukin 4 (IL-4), welches die Differenzierung von TH0-Zellen zu TH2-Zellen und die klonale Vermehrung bewirkt. In Folge synthetisieren TH2-Zellen die typischen Zytokine IL-4 und IL-13, die wiederum die Differenzierung von naiven B-Zellen zu IgG4/IgE produzierenden Memory B-Zellen und deren klonale Expansion verursachen. AD-Patienten haben vermehrt aktivierte T-Zellen im Blut. Die zirkulierenden T-Zellen sind zu großen Anteilen CD4+ T-Zellen, die Anzahl der CD8+ Zellen ist nicht erhöht oder sogar leicht verringert.

Die Induktion der Zytokine IL-4, IL-13 aber auch IL-31 führt zur Herunterregulation der Expression von Filaggrin und des Keratinozytenproteins Loricin, welches die Korneozytenfunktion und die Hautbarriere weiter beeinträchtigt.

Das dermale Mikrobiom interagiert mit Immunzellen der Haut und moduliert so die Funktion des dermalen Immunsystems. Ebenso wie andere entzündliche Hauterkrankungen wie Akne ist auch die AD assoziiert mit einer dermalen Dysbiose. So lassen sich im akuten Schub eine deutlich reduzierte Diversität und eine veränderte Speziesverteilung des dermalen Mikrobioms nachweisen, welches dann häufig von Staphylococcus aureus dominiert wird. Dabei korreliert der Grad der Besiedlung mit dem Schweregrad der Erkrankung (Geoghegan et al., Staphylococcus aureus and Atopic Dermatitis: A Complex and Evolving Relationship, Trends Microbiol., 2018; 26(6): 484-97).

Die atopische Dermatitis hat eine hohe Prävalenz und ist in vielen Fällen mit einer hohen Einschränkung der Lebensqualität verbunden. Bei vielen Patienten bleibt die Erkrankung über das Kindesalter hinaus bestehen, was eine langfristige Therapie erforderlich macht.

Insbesondere vor diesem Hintergrund ist die Suche nach nebenwirkungsarmen, innovativen Therapieformen und unterstützenden Maßnahmen dringend notwendig. Dabei stellen Probiotika einen vielversprechenden Ansatz dar, denn ebenso wie andere entzündliche Hauterkrankungen wie Akne ist auch die atopische Dermatitis assoziiert mit einer dermalen, aber auch intestinalen Dysbiose.

Zur oralen Anwendung von Prä-, Pro- und Synbiotika wurden in den letzten Jahren vermehrt Studien durchgeführt, die Metaanalysen zufolge überwiegend positive Effekte gezeigt haben.

Die lokale Anwendung probiotischer Präparate ist bisher weit weniger gut untersucht. In einer open-label Phase I/II Studie konnte gezeigt werden, dass die lokale Applikation von Roseomonas mucosa isoliert von gesunden Spendern eine signifikante Verbesserung der Symptomatik bewirken konnte. Auch die Notwendigkeit der Anwendung von Steroiden und die Besiedlung der Haut mit S. aureus konnte reduziert werden (Myles et al., First-in-human topical microbiome transplantation with Roseomonas mucosa for atopic dermatitis, JCI Insight, 2018, 3(9)).

In einer einfach verblindeten, randomisierten, placebo-kontrollierten Studie wurde die lokale Anwendung eines probiotischen Nahrungsergänzungsmittels untersucht, welches als Bad bzw. Teilbad angewendet wurde. Die Studienteilnehmer beurteilten den allgemeinen Hautzustand sowie die Symptome Juckreiz, Schuppung, Trockenheit der Haut und Ausprägung von Rhagaden/Exkoriationen. Darüber hinaus bewerteten die Studienteilnehmer die Einschränkung in ihrem Alltag, die sich aus der Erkrankung ergibt. Für alle Parameter konnte nach 7 Tagen, sowie über den gesamten Behandlungszeitraum von 14 Tagen eine Verbesserung verzeichnet werden. Allerdings wurde festgestellt, dass noch weitere Studien notwendig sind, um geeignete Bakterienstämme zu identifizieren und mehr Informationen über Dosierung und Wirkmechanismen zu erhalten (Axt-Gadermann, Significant Improvement of Skin Conditions in Atopic Dermatitis by Synbiotic Bath Additives, Akt Dermatol, 2018, 44: 1-8.).

Die EP 2 228 067 A1 offenbart eine pharmazeutische Zusammensetzung zur Verwendung in der Vorbeugung und Behandlung von Neurodermitis, die probiotische Mikroorganismen aus der Gruppe bestehend aus Lactobacillus, Bifidobacterium und Streptococcus, und darüber hinaus auch eine präbiotische Verbindung enthält.

In der EP 3 484 585 B1 wird ebenfalls eine probiotische Zusammensetzung mit Bakterien der Gattungen Lactobacillus und Bifidobacter beschrieben, die zur Behandlung von Neurodermitis verwendet werden und zusätzlich Maltodextrin enthalten kann.

Die EP 3 176 175 A1 offenbart Zusammensetzungen, die L-Arabinogalactoside und probiotische Bakterienstämme wie Bifidobakterien beinhalten und ebenfalls zur Behandlung von Neurodermitis verwendet werden können.

Die BR 1020 1900 6837 A2 beschreibt eine kosmetische Zusammensetzung zur Behandlung von Neurodermitis umfassend Lactobacillen.

Die WO 2016/149687 A1 offenbart eine pharmazeutische Zusammensetzung zur topischen Anwendung zur Behandlung von Neurodermitis. Die Zusammensetzung kann Lactobacillus und Streptococcus und Präbiotika wie Pektin oder Inulin enthalten.

Die WO 2019/180748 A1 beschreibt eine probiotische Formulierung zur topischen Anwendung als Anti-Akne Mittel. Diese werden in einer Konzentration zwischen 10⁶ kbE bis 10¹⁴ kbE eingesetzt.

Die CN 111 374 896 A offenbart weiters eine präbiotische und probiotische Zusammensetzung, zur Behandlung von Akne. Die Zusammensetzung enthält Bakterienstämme der Gattungen Bifidobacterium, Lactobacillus und Streptococcus und eine präbiotische Komponente, wie zum Beispiel Inulin.

Die CN 104 839 654 A betrifft eine Zusammensetzung, die ein Präbiotikum wie Inulin oder Stärke, sowie ein Probiotikum aus der Gattung Bifidobakterium und Lactobacillus enthaltet und ebenfalls zur Behandlung von Akne verwendet werden kann.

Die CN 111 568 846 A beschreibt Inulin als Präbiotikum zur Behandlung von Akne.

Die WO 2020/234867 A1 offenbart Zusammensetzungen, die zur Behandlung von Akne und Neurodermitis verwendet werden können, und probiotische Mikroorganismen wie Lactobacillus, Bifidobacterium und Streptococcus umfassen.

Die WO 2020/120670 A1 betrifft eine topische Formulierung ebenfalls zur Behandlung von Akne oder Neurodermitis, die probiotische Bakterien aus der Gattung Lactobacillus, Bifidobacterium oder Streptococcus enthalten kann.

Somit besteht ein Bedarf an der Entwicklung von topisch anwendbaren, pro-, prä- und synbiotischen Zusammensetzungen, die zur Behandlung von Erkrankungen der Haut verwendet werden können. Dementsprechend ist die Aufgabe der vorliegenden Erfindung die Bereitstellung einer pharmazeutischen Zusammensetzung, die zur Verwendung bei der Behandlung von Hauterkrankungen wie Neurodermitis oder Akne eingesetzt werden kann und zur erheblichen Verbesserung der jeweiligen Symptomatik führt.

### Zusammenfassung der Erfindung

Die oben genannte Aufgabe wird durch die vorliegende Erfindung, wie sie in den anhängigen Ansprüchen 1-7 definiert wird, gelöst.

Die vorliegende Erfindung bezieht sich auf eine pharmazeutische Zusammensetzung zur Verwendung bei der Behandlung von Neurodermitis, umfassend ein Probiotikum, wobei das Probiotikum die Mikroorganismen Lactobacillus paracasei, Bifidobacterium longum, Streptococcus thermophilus, Lactobacillus johnsonii, Lactobacillus reuteri, Lactobacillus plantarum, Lactobacillus gasseri, Lactobacillus rhamnosus und Bifidobacterium lactis umfasst.. Ferner kann die Gesamtkonzentration der Mikroorganismen in der Zusammensetzung im Bereich von 0,2 x 10⁹ KbE/g bis 3,0 x 10⁹ KbE/g (KbE = koloniebildende Einheiten), optional im Bereich von 0,6 x 10⁹ KbE/g bis 2,4 x 10⁹ KbE/g, ferner optional im Bereich von 1,0 x 10⁹ KbE/g bis 2,0 x 10⁹ KbE/g liegen oder ungefähr 1,8 x 10⁹ KbE/g sein.

Weiterhin kann die pharmazeutische Zusammensetzung ein Präbiotikum umfassen, das vorzugsweise aus der Gruppe bestehend aus Inulin, Maltodextrin, Pektin und resistente Stärke ausgewählt ist. Besonders bevorzugt ist das Präbiotikum der pharmazeutischen Zusammensetzung Inulin und/oder Maltodextrin.

Offenbart ist auch eine pharmazeutische Zusammensetzung, umfassend ein Synbiotikum, wobei das Synbiotikum ein wie vorstehend definiertes Probiotikum und ein wie vorstehend definiertes Präbiotikum umfasst.

Die vorstehend genannten pharmazeutischen Zusammensetzungen können als topische Darreichungsformen, insbesondere als Creme, Salbe oder Öl, oder als Badezusatz vorliegen.

Die Erfindung bezieht sich weiterhin auf ein Wasserbad, umfassend 0,5 g bis 10 g, optional 1,0 g bis 8,0 g, ferner optional 2,0 g bis 6,0 g, oder optional 2,5 g oder 5,0 g einer wie vorstehend definierten pharmazeutischen Zusammensetzungen pro Liter Wasser.

Außerdem wird ein Verfahren zur Herstellung des Wasserbades bereitgestellt, umfassend das Mischen der vorstehend definierten pharmazeutischen Zusammensetzung mit Wasser, so dass das Bad eine Konzentration der Zusammensetzung von 0,5 g bis 10 g pro Liter Wasser aufweist.

Ferner kann die vorstehend definierte pharmazeutische Zusammensetzung oder das vorstehend definierte Wasserbad bei der Behandlung von Neurodermitis verwendet werden.

Offenbart ist auch, dass die vorstehend definierte pharmazeutische Zusammensetzung bei der Behandlung von Neurodermitis verwendet werden kann, wobei die Mikroorganismen ausgewählt sind aus der Gruppe bestehend aus Bifidobacterium bifidum, Bifidobacterium breve, Bifidobacterium lactis, Bifidobacterium longum, Bifidobacterium longum subsp. Infantis, Lactobacillus johnsonii, Lactobacillus acidophilus, Lactobacillus brevis, Lactobacillus bulgaricus, Lactobacillus casei, Lactobacillus fermentum, Lactobacillus gasseri, Lactobacillusparacasei, Lactobacillus plantarum, Lactobacillus reuteri, Lactobacillus rhamnosus (ATCC 53103), Lactobacillus rhamnosus, Lactobacillus salivariusLs, Lactococcus lactis, Streptococcus thermophilus, Streptococcus salivarius, Enterococcus faecalis, Enterococcus faecium, Saccharomyces boulardii, Saccharomyces cerevisiae und/oder E. coli. Ferner können die Mikroorganismen ausgewählt sein aus der Gruppe bestehend aus Lactobacillus paracasei, Bifidobacterium longum, Streptococcus thermophilus, Lactobacillus johnsonii, Lactobacillus reuteri, Lactobacillus plantarum, Lactobacillus gasseri, Lactobacillus rhamnosus und Bifidobacterium lactis.

Offenbart ist auch, dass die vorstehend definierte pharmazeutische Zusammensetzung bei der Behandlung von Akne verwendet werden kann, wobei die Mikroorganismen ausgewählt sind aus der Gruppe bestehend aus Streptococcus salivarius, Lactococcus spp., Streptococcus thermophilus, Lactobacillus delbrueckii, Lactobacillus plantarum, Lactobacillus paracasei, Lactobacillus acidophilus, Bifidobacterium adolescentis, und/oder Bifidobacterium animalis.

### Beschreibung der Figuren

Bei allen Figuren wurden die Mittelwerte mit One-Way-ANOVA verglichen und signifikante Veränderungen über die Zeit (p<0.05) mit einem Sternchen gekennzeichnet. Die Kennwerte der deskriptiven Statistik sind den jeweiligen Tabellen im Ausführungsbeispiel zu entnehmen.
Figur 1: Darstellung des SCORADs an Tag 0, 7 und 14 für die Behandlungsgruppen, die ein Teilbad in niedriger Dosierung (A) oder hoher Dosierung (B) erhalten haben, bzw. für die Gesamtpopulation (C).
Figur 2: Darstellung des local SCORADs an Tag 0, 7 und 14 für die Behandlungsgruppen, die ein Teilbad in niedriger Dosierung (A) oder hoher Dosierung (B) erhalten haben, bzw. für die 25 Gesamtpopulation (C).
Figur 3: Beurteilung des Symptoms Erythem an Tag 0, 7 und 14 für die Behandlungsgruppen, die ein Teilbad in niedriger Dosierung (A) oder hoher Dosierung (B) erhalten hatten bzw. für die Gesamtpopulation (C).
Figur 4: Beurteilung des Symptoms Exkoriation an Tag 0, 7 und 14 für die Behandlungsgruppen, die ein Teilbad in niedriger Dosierung (A) oder hoher Dosierung (B) erhalten hatten bzw. für die Gesamtpopulation (C).
Figur 5: Beurteilung des Symptoms Ödeme an Tag 0, 7 und 14 für die Behandlungsgruppen, die ein Teilbad in niedriger Dosierung (A) oder hoher Dosierung (B) erhalten hatten bzw. für die Gesamtpopulation (C).
Figur 6: Beurteilung des Symptoms Krustenbildung an Tag 0, 7 und 14 für die Behandlungsgruppen, die ein Teilbad in niedriger Dosierung (A) oder hoher Dosierung (B) erhalten hatten bzw. für die Gesamtpopulation (C).
Figur 7: Beurteilung des Symptoms Lichenifikation an Tag 0, 7 und 14 für die Behandlungsgruppen, die ein Teilbad in niedriger Dosierung (A) oder hoher Dosierung (B) erhalten hatten bzw. für die Gesamtpopulation (C).
Figur 8: Beurteilung des Symptoms Trockenheit an Tag 0, 7 und 14 für die Behandlungsgruppen, die ein Teilbad in niedriger Dosierung (A) oder hoher Dosierung (B) erhalten hatten bzw. für die Gesamtpopulation (C).
Figur 9: Teilnehmerfragebogen: Bewertung der Hautzustandes an Tag 0, 7 und 14 für die Behandlungsgruppen, die ein Teilbad in niedriger Dosierung (A) oder hoher Dosierung (B) erhalten hatten bzw. für die Gesamtpopulation (C).
Figur 10: Teilnehmerfragebogen: Bewertung der Rötung der betroffenen Hautstellen an Tag 0, 7 und 14 für die Behandlungsgruppen, die ein Teilbad in niedriger Dosierung (A) oder hoher Dosierung (B) erhalten hatten bzw. für die Gesamtpopulation (C).
Figur 11: Teilnehmerfragebogen: Bewertung der Schuppung der betroffenen Hautstellen an Tag 0, 7 und 14 für die Behandlungsgruppen, die ein Teilbad in niedriger Dosierung (A) oder hoher Dosierung (B) erhalten hatten bzw. für die Gesamtpopulation (C).
Figur 12: Teilnehmerfragebogen: Bewertung der Trockenheit der betroffenen Hautstellen an Tag 0, 7 und 14 für die Behandlungsgruppen, die ein Teilbad in niedriger Dosierung (A) oder hoher Dosierung (B) erhalten hatten bzw. für die Gesamtpopulation (C).
Figur 13: Teilnehmerfragebogen: Bewertung des Juckreizes an Tag 0, 7 und 14 für die Behandlungsgruppen, die ein Teilbad in niedriger Dosierung (A) oder hoher Dosierung (B) erhalten hatten bzw. für die Gesamtpopulation (C).
Figur 14: Teilnehmerfragebogen: Bewertung der Einschränkungen im Alltag an Tag 0, 7 und 14 für die Behandlungsgruppen, die ein Teilbad in niedriger Dosierung (A) oder hoher Dosierung (B) erhalten hatten bzw. für die Gesamtpopulation (C).
Figur 15: Teilnehmerfragebogen: Bewertung der Schlafstörungen an Tag 0, 7 und 14 für die Behandlungsgruppen, die ein Teilbad in niedriger Dosierung (A) oder hoher Dosierung (B) erhalten hatten bzw. für die Gesamtpopulation (C).
Figur 16: SCORAD (A) localSCORAD (B) für die Subgruppe Kinder an Tag 0, 7 und 14.
Figur 17: 21 Teilnehmerfragebogen: Bewertung des allgemeinen Hautzustandes (A), der Trockenheit (B), der Schuppung (C) sowie der Rötung (D) an Tag 0, 7 und 14 für die Subgruppe Kinder.
Figur 18: Quantifizierung der Genkopienzahl von S. aureus an Tag 0, 7 und 14 für die Behandlungsgruppen, die ein Teilbad in niedriger Dosierung (A) oder hoher Dosierung (B) erhalten hatten bzw. für die Gesamtpopulation (C).
Figur 19: Quantifizierung der Genkopienzahl von S. aureus an Tag 0, 7 und 14 für die Subgruppe Kinder, die ein Teilbad in niedriger Dosierung (A) oder hoher Dosierung (B) erhalten hatten bzw. für die Gesamtpopulation (C).
Figur 20: Korrespondenzanalyse der bakteriellen Gemeinschaftsstrukturen von Hautabstrichen der beiden Behandlungsgruppen sowie der Gesamtpopulation als Mittelwerte der jeweiligen Zeitpunkte mit Standardfehler.

### Detaillierte Beschreibung der Erfindung

So nicht anderweitig definiert haben hierin verwendete technische und wissenschaftliche Ausdrücke dieselbe Bedeutung, wie sie von einem Fachmann auf dem Fachgebiet der Erfindung gemeinhin verstanden wird.

Präbiotika sind unverdauliche Nahrungsbestandteile, die Aktivität und Wachstum einzelner oder mehrerer Mikroorganismen günstig beeinflussen und damit die Gesundheit des Wirtes verbessern.

Probiotika sind lebende Mikroorganismen, die alleine oder als Zusatz in Nahrungsmitteln und Nahrungsergänzungsmitteln gesundheitsförderliche Effekte ausüben, die über das Maß grundlegender ernährungsphysiologischer Effekte hinausgehen.

Synbiotika sind Produkte, die sowohl Prä- als auch Probiotika enthalten. Die zugesetzten präbiotischen Ballaststoffe sollen das Wachstum und Überleben der probiotischen Keime sicherstellen.

Ballaststoffe bestehen aus Kohlenhydratpolymeren mit zehn oder mehr Monomereinheiten, die von den körpereigenen Enzymen im Dünndarm des Menschen nicht hydrolysiert werden.

Die Erfinder haben herausgefunden, dass die topische Anwendung eines Probiotikums, welches die Kombination von Mikroorganismen der bakteriellen Gattung Streptococcus mit Mikroorganismen der Gattung Lactobacillus und/oder Bifidobacterium umfasst, zu signifikanten Verbesserungen der Symptomatik von Hautkrankheiten wie Neurodermitis führt. Dies ist darauf zurückzuführen, dass bei Neurodermitis oftmals ein Mangel an Ceramiden besteht. Mikroorganismen der bakteriellen Gattung Streptococcus, insbesondere die Bakterienart Streptococcus thermophilus, sind durch den Besitz einer aktiven "neutralen Sphingomyelinase" in der Lage den Ceramidgehalt der Epidermis zu erhöhen.

Ferner kann die Gesamtkonzentration der Mikroorganismen in der Zusammensetzung im Bereich von 0,2 x 10⁹ KbE/g bis 3,0 x 10⁹ KbE/g, optional im Bereich von 0,6 x 10⁹ KbE/g bis 2,4 x 10⁹ KbE/g und ferner optional im Bereich von 1,0 x 10⁹ KbE/g bis 2,0 x 10⁹ KbE/g liegen. Weiterhin kann die Gesamtkonzentration der Mikroorganismen in der pharmazeutischen Zusammensetzung kann ungefähr 1,8 x 10⁹ KbE/g sein. Die Erfinder konnten zeigen, dass bei Anwendung der pharmazeutischen Zusammensetzung mit Mikroorganismen in diesem Konzentrationsbereich die Symptome der Hautkrankheiten erheblich gelindert werden konnten.

Die Erfindung bezieht sich auf eine pharmazeutische Zusammensetzung zur Verwendung bei der Behandlung von Neurodermitis, wobei die pharmazeutische Zusammensetzung ein Präbiotikum umfassen kann, das aus der Gruppe bestehend aus Inulin, Maltodextrin, Pektin und resistente Stärke ausgewählt ist. Die Ballaststoffe können dabei gewichtsmäßig zu gleichen Teilen enthalten sein. Die bereits auf der Haut vorhandenen Mikroorganismen können durch den Einsatz des Präbiotikums vermehrt werden.

Ferner kann das Präbiotikum der pharmazeutischen Zusammensetzung Inulin und/oder Maltodextrin sein.

Offenbart ist auch eine pharmazeutische Zusammensetzung, welche ein Synbiotikum umfasst, wobei das Synbiotikum ein wie vorstehend definiertes Probiotikum und ein wie vorstehend definiertes Präbiotikum umfasst. Dabei umfasst das Präbiotikum Mikroorganismen, die aus den bakteriellen Gattungen Lactobacillus und/oder Bifidobacterium, und Streptococcus ausgewählt sind. Die Gesamtkonzentration der Mikroorganismen in der Zusammensetzung kann im Bereich von 0,2 x 10⁹ KbE/g bis 3,0 x 10⁹ KbE/g, optional im Bereich von 0,6 x 10⁹ KbE/g bis 2,4 x 10⁹ KbE/g und ferner optional im Bereich von 1,0 x 10⁹ KbE/g bis 2,0 x 10⁹ KbE/g liegen oder ungefähr 1,8 x 10⁹ KbE/g sein. Das Präbiotikum ist aus der Gruppe bestehend aus Inulin, Maltodextrin, Pektin, resistente Stärke und/oder artverwandten Ballaststoffen ausgewählt oder kann Inulin und/oder Maltodextrin sein. Gewichtsmäßig kann das Präbiotikum der Hauptbestandteil der pharmazeutischen Zusammensetzung und/oder des Synbiotikums sein. Ferner kann das Präbiotikum in einer Menge von in etwa 98 Gewichts-% bezogen auf das Gesamtgewicht der pharmazeutischen Zusammensetzung enthalten sein. Weiterhin kann das Präbiotikum in einer Menge von mindestens 98 Gewichts-% bezogen auf das Gesamtgewicht der pharmazeutischen Zusammensetzung enthalten sein. Dabei kann das Präbiotikum aus Inulin, Maltodextrin, Pektin, resistenter Stärke und/oder artverwandten Ballaststoffen bestehen oder diese umfassen, wobei diese gewichtsmäßig zu jeweils in etwa gleichen Teilen enthalten sein können. Ferner kann das Präbiotikum aus gewichtsmäßig jeweils in etwa gleichen Teilen Inulin und Maltodextrin zusammengesetzt sein, sodass die Menge an Inulin und Maltodextrin jeweils in etwa 49 Gewichts-% bezogen auf das Gesamtgewicht der pharmazeutischen Zusammensetzung betragen kann. Weiterhin kann die Menge an Inulin und Maltodextrin jeweils mindestens 49 Gewichts-% bezogen auf das Gesamtgewicht der pharmazeutischen Zusammensetzung betragen. Das Präbiotikum kann dabei nicht nur den bereits auf der Haut befindlichen Bakterien, sondern auch den in der pharmazeutischen Zusammensetzung befindlichen Probiotika als Nährstoffquelle dienen.

Die vorstehend genannten pharmazeutischen Zusammensetzungen können als topische Darreichungsformen, insbesondere als Creme, Salbe oder Öl, oder als Badezusatz vorliegen.

Ein weiterer Aspekt der Erfindung bezieht sich auf ein Wasserbad, welches 0,5 g bis 10 g, optional 1,0 g bis 8,0 g, ferner optional 2,0 g bis 6,0 g, oder optional 2,5 g oder 5,0 g einer wie vorstehend definierten pharmazeutischen Zusammensetzungen pro Liter Wasser enthält. Die pharmazeutische Zusammensetzung umfasst dabei ein wie vorstehend definiertes Pro-, Prä- oder Synbiotkium.

Außerdem wird ein Verfahren zur Herstellung des Wasserbades bereitgestellt, umfassend das Mischen der vorstehend definierten pharmazeutischen Zusammensetzung mit Wasser, so dass das Bad eine Konzentration der Zusammensetzung von 0,5 g bis 10 g pro Liter Wasser aufweist. Optional kann die Zielkonzentration der Zusammensetzung 1,0 g bis 8,0 g, ferner optional 2,0 g bis 6,0 g, oder optional 2,5 g oder 5,0 g aufweisen.

Zur Anwendung des Wasserbades werden die betroffenen Körperteile in das Wasserbad gehalten, sodass die betroffenen Körperteile vollständig durch das Wasserbad bedeckt sind. Die Badedauer beträgt 10 Minuten. Anschließend wird die Körperstelle an der Luft getrocknet.

Ein weiterer Aspekt der Erfindung ist die Verwendung der pharmazeutischen Zusammensetzung oder des vorstehend definierten Wasserbads bei der Behandlung von Neurodermitis. Die pharmazeutische Zusammensetzung umfasst dabei ein wie vorstehend definiertes Pro-, Prä- oder Synbiotkium. Die Erfinder konnten zeigen, dass sich durch die Verwendung der pharmazeutischen Zusammensetzung der allgemeinen Hautzustand sowie Symptome wie Rötung, Schuppung, Juckreiz, Trockenheit, Erytheme, Exkoriation, Ödeme/Papelbildung, Lichenifikation sowie Nässen/Krustenbildung eindeutig verbessern lassen. Auch führt die Verwendung der erfindungsgemäßen pharmazeutischen Zusammensetzung zu einer Verringerung der Einschränkungen im Alltag sowie einer Besserung der Schlafstörungen durch die Hautveränderungen.

Offenbart ist auch, dass die vorstehend definierte pharmazeutische Zusammensetzung bei der Behandlung von Neurodermitis verwendet werden kann, wobei die Mikroorganismen ausgewählt sind aus der Gruppe bestehend aus Bifidobacterium bifidum, Bifidobacterium breve, Bifidobacterium lactis, Bifidobacterium longum, Bifidobacterium longum subsp. Infantis, Lactobacillus johnsonii, Lactobacillus acidophilus, Lactobacillus brevis, Lactobacillus bulgaricus, Lactobacillus casei, Lactobacillus fermentum, Lactobacillus gasseri, Lactobacillus paracasei, Lactobacillus plantarum, Lactobacillus reuteri, Lactobacillus rhamnosus (ATCC 53103), Lactobacillus rhamnosus, Lactobacillus salivariusLs, Lactococcus lactis, Streptococcus thermophilus, Streptococcus salivarius, Enterococcus faecalis, Enterococcus faecium, Saccharomyces boulardii, Saccharomyces cerevisiae und/oder E. coli. Ferner können die Mikroorganismen ausgewählt sein aus der Gruppe bestehend aus Lactobacillus paracasei, Bifidobacterium longum, Streptococcus thermophilus, Lactobacillus johnsonii, Lactobacillus reuteri, Lactobacillus plantarum, Lactobacillus gasseri, Lactobacillus rhamnosus und Bifidobacterium lactis. Die Gesamtkonzentration der Mikroorganismen in der Zusammensetzung kann im Bereich von 0,2 x 10⁹ KbE/g bis 3,0 x 10⁹ KbE/g, optional im Bereich von 0,6 x 10⁹ KbE/g bis 2,4 x 10⁹ KbE/g und ferner optional im Bereich von 1,0 x 10⁹ KbE/g bis 2,0 x 10⁹ KbE/g liegen oder ungefähr 1,8 x 10⁹ KbE/g sein. Dabei kann die pharmazeutische Zusammensetzung weiterhin ein Präbiotikum enthalten, wobei das Präbiotikum aus der Gruppe bestehend aus Inulin, Maltodextrin, Pektin, resistente Stärke und/oder artverwandten Ballaststoffen ausgewählt ist oder Inulin und/oder Maltodextrin ist. Gewichtsmäßig kann das Präbiotikum der Hauptbestandteil der pharmazeutischen Zusammensetzung sein. Ferner kann das Präbiotikum in einer Menge von in etwa 98 Gewichts-% bezogen auf das Gesamtgewicht der pharmazeutischen Zusammensetzung enthalten sein. Weiterhin kann das Präbiotikum in einer Menge von mindestens 98 Gewichts-% bezogen auf das Gesamtgewicht der pharmazeutischen Zusammensetzung enthalten sein. Dabei kann das Präbiotikum aus Inulin, Maltodextrin, Pektin, resistente Stärke und/oder artverwandten Ballaststoffen bestehen, wobei diese gewichtsmäßig zu jeweils in etwa gleichen Teilen enthalten sein können. Ferner kann das Präbiotikum aus gewichtsmäßig jeweils in etwa gleichen Teilen Inulin und Maltodextrin zusammengesetzt sein, sodass die Menge an Inulin und Maltodextrin jeweils in etwa 49 Gewichts-% bezogen auf das Gesamtgewicht der pharmazeutischen Zusammensetzung betragen kann. Weiterhin kann die Menge an Inulin und Maltodextrin jeweils mindestens 49 Gewichts-% bezogen auf das Gesamtgewicht der pharmazeutischen Zusammensetzung betragen. Die pharmazeutische Zusammensetzung kann ferner zur Herstellung eines wie vorstehend definierten Wasserbads eingesetzt werden.

Offenbart ist auch, dass die vorstehend definierte pharmazeutische Zusammensetzung bei der Behandlung von Akne verwendet werden kann, wobei die Mikroorganismen ausgewählt sind aus der Gruppe bestehend aus Streptococcus salivarius, Lactococcus spp., Streptococcus thermophilus, Lactobacillus delbrueckii, Lactobacillus plantarum, Lactobacillus paracasei, Lactobacillus acidophilus, Bifidobacterium adolescentis, und/oder Bifidobacterium animalis. Diese Mikroorgansimen sind insbesondere gegen P. acnes/Cutibacterium acnes wirksam und/oder weisen entzündungshemmende Effekte auf.

### Ausführungsbeispiel

Es wurde eine zwei-armige, randomisierte und verblindete Studie durchgeführt.

### 1. Übersicht über den Prüfplan

Die Studie umfasste vier Visiten, eine optionale "Auswaschphase" von 7 Tagen sowie einen Anwendungszeitraum von 14 Tagen.

Bei der ersten Visite (V1) erfolgte das Screening der Studienteilnehmer. Nach Aufnahme des Teilnehmers in die Studie folgte die siebentägige Auswaschphase - in welcher keine externen oder internen Therapeutika mehr appliziert werden durften. Befand sich der Teilnehmer zu V1 nicht unter Medikation, so konnten die Untersuchungen von Visite 2 direkt durchgeführt werden.

Visite 2 (V2) entsprach dem Zeitpunkt Tag 0. Es erfolgte die Bestimmung der Symptomatik durch den Arzt mittels des validierten Symptomscores SCORAD (SCORing Atopic Dermatitis) sowie durch den Studienteilnehmer mittels Fragebogen. Außerdem wurden oberflächliche Hautabstriche für die mikrobiologische Untersuchung genommen, sowie die Prüfprodukte ausgehändigt und deren Anwendung erläutert. Visite 3 (V3) und Visite 4 (V4) erfolgten an Tag 7 (+/- 1 Tag) sowie an Tag 14 (+/- 1 Tag). Es wurden dieselben Untersuchungen durchgeführt und Parameter erhoben wie zu V2.

### 1.1 Visiten und Untersuchungen

### Visite 1 (V1) - Screening

a. Aufklärung des Studienteilnehmers
b. Unterzeichnung der Einverständniserklärung des Probanden zur Teilnahme an der Studie
c. Überprüfung der Ein- und Ausschlusskriterien einschließlich symptombezogener Untersuchung und Erhebung des SCORADs
d. Dokumentation der medizinischen Vorgeschichte, Begleiterkrankungen und Begleitmedikation
e. Dokumentation der demographischen Daten
f. Aushändigung der Prüfprodukte und Anleitung zur Anwendung

### Visite 2 (V2) - Tag 0

a. Aushändigung und Ausfüllen des Teilnehmerfragebogens 1. Der Studienteilnehmer bewertet seinen allgemeinen Hautzustand sowie die Symptome Rötung, Schuppung, Juckreiz und Trockenheit. Darüber hinaus werden Einschränkungen im Alltag und Schlafstörungen durch die Hautveränderungen beurteilt. Alle Parameter werden auf einer Skala von 0-10 bewertet.
b. Erhebung des SCORADs durch den Prüfarzt.
c. Hautabstrich für eine anschließende, mikrobiologische Untersuchung. Der Hautabstrich wird mit dem Beprobungssystem VWR Transport Swabs Amies der Firma Mibius durchgeführt. Dieses wird direkt vor der Verwendung mit nuklease-freiem Wasser angefeuchtet. Anschließend wird mit wenig Druck für 30 Sekunden über das Ekzem gestrichen. Das Beprobungssystem wird anschließend bei -20°C eingefroren.

### Visite 3 (V3) Tag 7 (± 1)

a. Aushändigung und Ausfüllen des Teilnehmerfragebogens 2. Der Studienteilnehmer bewertet seinen allgemeinen Hautzustand sowie die Symptome Rötung, Schuppung Juckreiz, Trockenheit. Darüber hinaus werden Einschränkungen im Alltag und Schlafstörungen durch die Hautveränderungen beurteilt. Alle Parameter werden auf einer Skala von 0-10 bewertet.
b. Erhebung des SCORADs durch den Prüfarzt.
c. Hautabstrich für eine anschließende, mikrobiologische Untersuchung. Der Hautabstrich wird mit dem Beprobungssystem VWR Transport Swabs Amies der Firma Mibius durchgeführt. Dieses wird direkt vor der Verwendung mit nuklease-freiem Wasser angefeuchtet. Anschließend wird mit wenig Druck für 30 Sekunden über das Ekzem gestrichen. Das Beprobungssystem wird anschließend bei -20°C eingefroren.

### Visite 4 (V4) Tag 14 (± 1)

a. Aushändigung und Ausfüllen des Teilnehmerfragebogens 3. Der Studienteilnehmer bewertet seinen allgemeinen Hautzustand sowie die Symptome Rötung, Schuppung Juckreiz und Trockenheit. Darüber hinaus werden Einschränkungen im Alltag und Schlafstörungen durch die Hautveränderungen beurteilt. Alle Parameter werden auf einer Skala von 0-10 bewertet.
b. Erhebung des SCORADs durch den Prüfarzt.
c. Hautabstrich für eine anschließende, mikrobiologische Untersuchung. Der Hautabstrich wird mit dem Beprobungssystem VWR Transport Swabs Amies der Firma Mibius durchgeführt. Dieses wird direkt vor der Verwendung mit nuklease-freiem Wasser angefeuchtet. Anschließend wird mit wenig Druck für 30 Sekunden über das Ekzem gestrichen. Das Beprobungssystem wird anschließend bei -20°C eingefroren.

### 1.2 Einschlusskriterien

- Studienteilnehmer müssen mindestens 5 Jahre alt sein. Bei Kindern und Jugendlichen unter 16 Jahren wird der Fragebogen von den Eltern bzw. zusammen mit den Eltern ausgefüllt.
- Unterzeichnete Einverständniserklärung zur Teilnahme an der Studie
- Atopische Ekzeme an Händen/Armen und Füßen/Beinen: SCORAD Ausgangswert > 10. Die Kriterien Erythem + Exkoriation oder Krustenbildung sollten als Marker für ein akutes Geschehen vorhanden sein.
- Wirkstoffhaltige Externa wurden mindestens 7 Tage vor Studienbeginn abgesetzt: Kortikosteroide, Immunsuppressiva wie Pimecrolimus oder Tacrolimus sowie desinfizierende oder antibiotische Externa (desinfizierende Bäder, Salben)

### 1.3 Ausschlusskriterien

- erforderliche systemische Therapie des atopischen Ekzems: Anwendung von Kortisonpräparaten, Antihistaminika
- Therapie mit Wirkstoffen, die das Mikrobiom der Haut beeinflussen können, wie Antibiotika
- Immunsuppression: Personen mit einer ausgeprägten Abwehrschwäche (nach Organ- oder Stammzelltransplantationen, unter Chemotherapie oder hochdosierter Kortisontherapie)
- Schwangerschaft und Stillzeit

### 2. Anwendung

Es wurde ein Badezusatz angewendet, der 9 probiotische Bakterienstämme enthält. Der Badezusatz wurde in zwei unterschiedlichen Dosierungen getestet.

### 2.1 Zusammensetzung des Badezusatzes ActivaDerm sowie des Bades

**Tabelle 1: Verwendete Bakterienstämme im Badezusatz**

| **Bakterienstamm** | **Koloniebildende Einheiten pro Gramm** |
|---|---|
| Lactobacillus plantarum | 0,2 x 10⁹ KbE/g |
| Lactobacillus gasseri | 0,2 x 10⁹ KbE/g |
| Lactobacillus rhamnosum | 0,2 x 10⁹ KbE/g |
| Lactobacillus paracasei | 0,2 x 10⁹ KbE/g |
| Bifidobacterium longum | 0,2 x 10⁹ KbE/g |
| Streptococcus thermophilus | 0,2 x 10⁹ KbE/g |
| Lactobacillus johnsonii | 0,2 x 10⁹ KbE/g |
| Lactobacillus reuteri | 0,2 x 10⁹ KbE/g |
| Bifidobacterium lactis | 0,2 x 10⁹ KbE/g |

Die Konzentration (Aktivität) beträgt insgesamt: 1,8 x 10⁹ KbE/g

Weitere Bestandteile: Inulin und Maltodextrin zu gewichtsmäßig jeweils gleichen Teilen.

### Dosierung

1) 5 Gramm pro Liter entsprechend 9 x 10⁹ KbE/Liter (je Stamm 1 x 10⁹ KbE/L)
2) 2,5 Gramm pro Liter entsprechend 4,5 x 10⁹ KbE/Liter (je Stamm 0,5 x10⁹ KbE/L)

Die betroffenen Hautstellen wurden täglich für 10 Minuten gebadet.

### 2.2 Randomisierung und Verblindung

Die Zuteilung der Studienteilnehmer erfolgte anhand einer Randomisierung. Die Verblindung wurde durch die nicht-unterscheidbare Verpackung der Prüfprodukte gewährleistet.

### 2.3 Anweisungen für den Studienteilnehmer zur Anwendung der Studienmedikation

Der Studienteilnehmer hat das probiotische Bad nicht im Prüfzentrum, sondern zu Hause selbst angewendet. Hierzu hat er eine ausführliche Anleitung erhalten, die folgende Informationen umfasst:
- Füllen Sie eine kleine Wanne, einen Eimer o.ä. soweit mit lauwarmem Wasser, dass die betroffenen Körperteile bedeckt sind (das Wasser sollte nicht zu heiß sein)
- Bitte füllen Sie das Wasser mit einem Messbecher ein, damit Sie wissen, wie viele Liter Wasser in Ihrem Bad sind
- Geben Sie pro Liter Wasser einen Messlöffel Pulver in das Bad und rühren Sie das Wasser mit einem Löffel gut um.
- Badedauer: 10 Minuten
- Lassen Sie anschließend die Körperstelle an der Luft trocknen, waschen Sie das Badewasser nicht ab.
- Wenn sich die Haut sehr trocken anfühlt, dann können Sie diese frühestens nach einer Stunde mit einer wirkstofffreien Basispflege eincremen. Sonst bitte nichts anwenden (kein Kortison, Protopic o.ä.).
- Sie dürfen frühestens acht Stunden nach der Anwendung des Bades duschen. Auch die Hände sollten Sie in dieser Zeit nicht waschen, falls diese mit dem probiotischen Bad behandelt werden. Es empfiehlt sich deshalb die Anwendung am Abend.
- Bitte füllen Sie täglich, nach der Anwendung des Bades, die beiliegende CHECKLISTE aus und geben Sie diese zum Abschluss der Studie beim Prüfarzt ab.

### 2.4 Lagerbedingungen

Der probiotische Badezusatz wurde bei Raumtemperatur in der Originalverpackung gelagert. Um die Aufnahme von Feuchtigkeit möglichst zu reduzieren, sollte die Verpackung nach jeder Entnahme sofort wieder fest verschlossen werden. Es sollte vermieden werden, dass der enthaltene Messlöffel mit dem Badewasser in Kontakt kommt.

### 2.5 Begleitmedikation während der Studie

Jegliche Begleitmedikation (einschließlich OTC-Präparaten, Nahrungsergänzungsmitteln und pflanzlichen Arzneimitteln), die während der Studie eingenommen wurde, wurde im Beobachtungsbogen dokumentiert.

### 2.6 Compliance

Es wurde anhand einer Checkliste für den Studienteilnehmer überprüft, ob er das Bad täglich angewendet hat. Darin wurden die tägliche Anwendung sowie evtl. auftretende Besonderheiten dokumentiert.

### 3. Untersuchung der klinischen Wirksamkeitsparameter

Die Veränderung der klinischen Symptomatik wurde anhand des SCORADs durch den Prüfarzt, sowie durch die Bewertung des Studienteilnehmers anhand eines Fragebogens analysiert.

### 3.1 SCORAD

Zur Bewertung wurde der validierte Symptomscore SCORAD verwendet (Severity scoring of atopic dermatitis: the SCORAD index. Consensus Report of the European Task Force on Atopic Dermatitis. Dermatology. 1993;186(1):23-31.).

### 3.2 Teilnehmerfragebogen

Im Teilnehmerfragebogen wurden folgende Parameter in einer numerischen Skala von 0-10 vom Studienteilnehmer dokumentiert:
- Bewertung des allgemeinen Hautzustandes
- Bewertung der Rötung
- Bewertung der Schuppung
- Bewertung des Juckreizes
- Bewertung der Trockenheit
- Einschränkungen im Alltag
- Schlafstörungen durch die Hautveränderungen

### 4. Mikrobiologische Untersuchungen

Da in der Literatur ein Zusammenhang zwischen der Schwere der Symptomatik der atopischen Dermatitis, der Diversität des bakteriellen Hautmikrobioms sowie der Quantität von S. aureus beschrieben ist, wurden Hautabstriche der Studienteilnehmer genommen. Der Hautabstrich wurde mit dem Beprobungssystem VWR Transport Swabs Amies der Firma Mibius durchgeführt. Die Beprobungssysteme wurden bei -20°C bis zur Nukleinsäureextraktion gelagert. Die Nukleinsäureextraktion mittels Phenol/Chloroform erfolgte mit wenigen Modifikationen nach der Veröffentlichung Noll et al. (Noll et al., Succession of bacterial community structure and diversity in a paddy soil oxygen gradient, Environ Microbiol, 2005, 7(3): 382-395).

### 4.1 Entnahme der Hautproben

Der Hautabstrich wurde mit dem Beprobungssystem VWR Transport Swabs Amies der Firma Mibius durchgeführt. Dieses wurde direkt vor der Verwendung mit nuklease-freiem Wasser angefeuchtet. Anschließend wurde mit wenig Druck für 30 Sekunden über das Ekzem gestrichen. Das Beprobungssystem wurde anschließend bei -20°C eingefroren und verblieb im Prüfzentrum bis zum gekühlten Versand zum mikrobiologischen Labor.

### 4.2 DNA-Isolation aus Hautproben

Im Folgenden sind die Modifikationen des Protokolls nach Noll et al. 2005 aufgelistet. Zu allen Proben wurde eine Spatelspitze Zirkoniumbeads (ca. 0,2 g), 400 µL kalter TPM-Puffer, 200 µL NaPO4- Puffer und 600 µL SDS-Aquaphenol-Gemisch gegeben. Anschließend wurden die Proben gevortext und bei 65°C für 10 Minuten inkubiert. Danach wurde jedes Gemisch im FastPrep 24 für 60s bei 4,5 m/s homogenisiert und danach bei -80°C für 5 Minuten eingefroren. Anschließend wurden die Proben bei 21.500 x g und 4°C für 15 Minuten zentrifugiert. 800 µL des Überstands wurde dasselbe Volumen an TPM-Puffer zugegeben, gevortext und erneut zentrifugiert (21.500 x g, 4°C, 15 Minuten). Das maximale Volumen an Überstand (ca. 800 µL) wurde abgenommen und mit dem gleichen Volumen von kaltem Phenol-Chlorofom-Isoamylalkohol vermischt und zentrifugiert (21.500 x g, 4°C, 15 Minuten). Zu 650 µL Überstand wurden 1300 µL PEG-Puffer sowie 2 µL Glycogen gegeben. Das Gemisch wurde gevortext und für 45 Minuten bei 4°C und 21.500 x g zentrifugiert. Der Überstand wurde verworfen. Das Nukleinsäure-Pellet wurde zweimal mit 500 µL Ethanol gewaschen und bei 37°C getrocknet. Das gereinigte DNA-Pellet wurde anschließend in 50 µL TE-Puffer resuspendiert.

Anschließend wurden die Quantität (A260) und Qualität (A260/280, A260/230) der DNA photometrisch mittels der µDropTM Plate nach Herstellerangaben bestimmt.

### 4.3 qPCR Staphylococcus aureus

Im Anschluss wurde eine quantitative polymerase chain reaction (qPCR) mithilfe des CFX96 Real-Time Systems von Biorad durchgeführt. Mithilfe der qPCR wurde die quantitative Veränderung (V2, V3 und V4) der Genkopienzahl von S. aureus ermittelt.

Zur Quantifizierung der Genkopienzahl wurde ein externer Standard von S. aureus (DSMZ 346), der zuvor von der Deutschen Stammsammlung von Mikroorganismen und Zellkulturen erworben wurde, generiert, sowie dies unter Noll et al., 2019 beschrieben worden ist (Noll et al., Copper containing wood preservatives shifted bacterial and fungal community compositions in pine sapwood in two field sites, Int Biodeter Biodegr, 2019, 142: 26-35). Mithilfe von Nukleinsäureextrakten mit definierter DNA-Konzentration und Gesamtzellzahl kann die Genkopienzahl der einzelnen Hautabstriche bestimmt werden. Der externe Standard wurde aus der Bakterienspezies S. aureus generiert. Hierzu wurde eine Übernachtkultur von S. aureus in BHI-Medium angesetzt. Die Zellzahl der Kultur wurde dann mittels Neubauer Zählkammer nach Nieschlag et al., 2012 bestimmt (Nieschlag E., WHO Laborhandbuch: Zur Untersuchung und Aufarbeitung des menschlichen Ejakulates, 2012, Springer-Verlag) und anschließend wurden aus einem Volumen von 500 µL der Kultur die Nukleinsäuren (siehe 4.3) extrahiert. Mit einer Verdünnungsreihe der Nukleinsäureextrakte (siehe 4.3) wurde die Genkopienzahl von S. aureus mittels qPCR im Abgleich mit der mikroskopisch gezählten Mikroorganismenanzahl ermittelt und als externer Quantifizierungsstandard verwendet. Das speziesspezifische qPCR Protokoll, anhand des nucA-Gens, wurde nach der Beschreibung von Brakstad et al., 1992 ohne Modifikationen durchgeführt (Brakstad, Aasbakk et al., Detection of Staphylococcus aureus by polymerase chain reaction amplification of the nuc gene, J Clin Microbiol, 1992, 30(7): 1654-1660). Als Polymerase wurde der iTaq Universal SYBR Green Supermix (Biorad^{®}) verwendet.

Das nucA-Gen und dessen Länge weisen eine hohe Spezifizät für die Bakterienart S. aureus auf. Das nucA-Gen stellt den Vorläufer der Thermonuclease der Spezies S. aureus dar. Durch das seltene Vorkommen des Sequenzmotifs des nucA-Gens bei anderen Bakterienspezies wird mit hoher Wahrscheinlichkeit bei einer qPCR nur die genomische DNA der Bakterienspezies S. aureus vervielfältigt (Barouei, Moussavi et al., Effect of maternal probiotic intervention on HPA axis, immunity and gut microbiota in a rat model of irritable bowel syndrome, PLoS One, 2012, 7(10): e46051). Auch das sequenzspezifische Primerpaar für S. aureus bindet mit hohen Spezifität das nucA-Fragment (Brakstad, Aasbakk et al., Detection of Staphylococcus aureus by polymerase chain reaction amplification of the nuc gene, J Clin Microbiol, 1992, 30(7): 1654-1660).

### 4.4 16S rRNA Gen- und ITS-spezifische Hochdurchsatzsequenzierung

Die bakterielle 16S rRNA Gen- und ITS (internal transcribed spacer region)-spezifische Hochdurchsatzsequenzierung wurde mittels Illumina MiSeq-Hochdurchsatzsequenzierung Technologie anhand der Veröffentlichung von Caproso et al., 2012 (Caporaso, Lauber et al., Ultra-high-throughput microbial community analysis on the Illumina HiSeq and MiSeq platforms., 2012, ISME J 6(8): 1621-1624) mit dem Primersatz von Noll et al. 2019 (Noll et al., Copper containing wood preservatives shifted bacterial and fungal community compositions in pine sapwood in two field sites, 2019, 142: 26-35) durchgeführt. Die Prozessierung der entstehenden Sequenzdaten und deren statistische Auswertung wurden nach Noll et al. 2019 (Noll et al., Copper containing wood preservatives shifted bacterial and fungal community compositions in pine sapwood in two field sites, 2019, 142: 26-35) durchgeführt.

### 5. Statistik

Alle statistischen Auswertungen wurden mit Hilfe des Statistikprogramms R der Version 3.5.2 beziehungsweise in R-Studio der Version 1.1.463 durchgeführt. Die Experimentaldaten des SCORADs wurden mittels Shapiro-Wilk-Test auf Normalverteilung getestet. Wird von einer Normalverteilung ausgegangen, wird, da die Proben unabhängig voneinander sind, die ANOVA ohne Verbindung zwischen den Stichproben gewählt. Anschließend wurde ein paarweiser t-Test verwendet, um zu ermitteln, zwischen welchen Gruppen der signifikante Unterschied besteht. Dabei wird der p-Wert einer Bonferroni-Korrektur unterzogen. Als Signifikanzniveau wurde α = 0,05 gewählt.

Beim Fehlen der Normalverteilung wurde über den Kruskal-Wallis-Test ein signifikanter Unterschied in den Mittelwerten gesucht. Mittels eines paarweisen Wilcoxon-Tests wurde anschließend überprüft, zwischen welchen Gruppen der signifikante Unterschied besteht. Dabei wurde der p-Wert einer Bonferroni-Korrektur unterzogen.

### 6. Ergebnisse

### 6.1 Bewertung durch den Prüfarzt

### 6.1.1 SCORAD

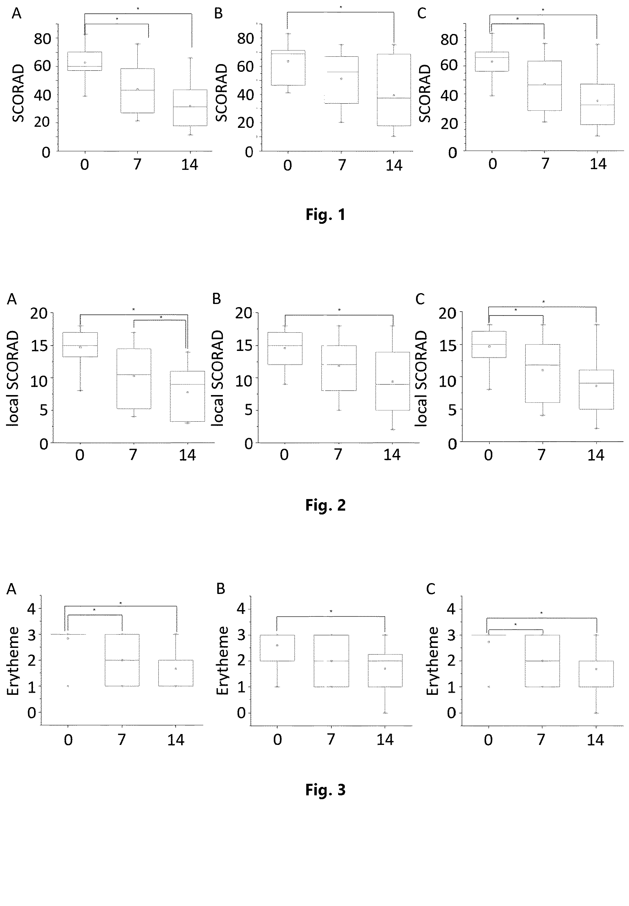

Die Auswertung des SCORADs zeigt, dass in beiden Behandlungsgruppen ein deutlicher Trend zur Verbesserung der klinischen Symptomatik während des Studienverlaufs besteht (siehe Figur 1 und Tabelle 2). Sowohl an Tag 7 als auch an Tag 14 sinkt der Mittelwert des SCORADs im Vergleich zum vorhergehenden Zeitpunkt. In der Behandlungsgruppe mit der niedrigeren Dosierung stellt sich dieser Trend etwas deutlicher dar; im Vergleich zum Ausgangswert an Tag 0 ist der Unterschied des SCORADs hier bereits an Tag 7 statistisch signifikant. In der Behandlungsgruppe mit der höheren Dosierung kann die statistische Signifikanz an Tag 14 festgestellt werden. Da anhand der Ergebnisse nicht von einem Unterschied in der Wirksamkeit beider Dosierungen ausgegangen werden kann, können alle Teilnehmer zur Gesamtpopulation entsprechend einer Dosierung von 4,5 bis 9 x 10⁹ KbE/L zusammengefasst werden. Hier ergibt sich eine signifikante Reduktion des mittleren SCORADs von 63,04 an Tag 0 auf 47,09 (-15,95) an Tag 7 und 35,26 (-27,78) an Tag 14.

**Tabelle 2: Ergebnisse der Analyse des SCORADs beider Behandlungsgruppen sowie der Gesamtpopulation**

| **SCORAD** | **A niedrige Dosis** | | | **B hohe Dosis** | | | **C Gesamtpopulation** | | |
|---|---|---|---|---|---|---|---|---|---|
| Tag | 0 | 7 | 14 | 0 | 7 | 14 | 0 | 7 | 14 |
| Mittelwert | **62,68** | **43,68** | **31,81** | **63,48** | **51,17** | **39,41** | **63,04** | **47,09** | **35,26** |
| Standardabweichung | 11,88 | 17,50 | 17,26 | 14,20 | 19,87 | 24,48 | 12,67 | 18,55 | 20,69 |
| Minimum | 38,90 | 21,40 | 11,40 | 41,30 | 20,30 | 10,50 | 38,90 | 20,30 | 10,50 |
| Unteres Quartil | 57,10 | 27,65 | 18,15 | 47,50 | 37,80 | 20,00 | 56,90 | 28,90 | 18,90 |
| Median | 60,10 | 43,10 | 31,25 | 68,80 | 55,95 | 37,35 | 65,85 | 46,60 | 32,25 |
| Oberes Quartil | 69,75 | 58,10 | 42,35 | 69,80 | 66,20 | 68,20 | 69,80 | 63,00 | 44,30 |
| Maximum | 82,80 | 75,80 | 65,80 | 83,10 | 75,30 | 75,30 | 83,10 | 75,80 | 75,30 |
| N (Anzahl) | 12 | 12 | 12 | 10 | 10 | 10 | 22 | 22 | 22 |

6.1.2 Teil B des SCORADs - local SCORAD

Im Teilabschnitt B des SCORADs wird die Intensität der Symptomatik der behandelten Hautstellen bewertet. Eine Teilauswertung dieses Abschnittes im Sinne eines local SCORADs ist daher zusätzlich aussagekräftig für den Therapieeffekt.

Die Ergebnisse stimmen hier überein mit den Ergebnissen des gesamten SCORADs (siehe Figur 2): Für die Gesamtpopulation sowie für die Behandlungsgruppe mit der niedrigeren Dosierung ist die Verbesserung signifikant an Tag 7 und an Tag 14 im Vergleich zum Ausgangswert an Tag 0. Für die Teilnehmergruppe mit der höheren Dosierung ist der Unterschied an Tag 14 signifikant (p<0,05).

**Tabelle 3: Ergebnisse der Analyse des localSCORADs beider Behandlungsgruppen sowie der Gesamtpopulation**

| **local SCORAD** | **A niedrige Dosis** | | | **B hohe Dosis** | | | **C Gesamtpopulation** | | |
|---|---|---|---|---|---|---|---|---|---|
| Tag | 0 | 7 | 14 | 0 | 7 | 14 | 0 | 7 | 14 |
| Mittelwert | **14,75** | **10,25** | **7,75** | **14,60** | **11,80** | **9,40** | **14,68** | **10,99** | **8,54** |
| Standardabweichung | 2,86 | 4,61 | 3,93 | 3,10 | 4,24 | 5,33 | 2,91 | 4,41 | 4,62 |
| Minimum | 8 | 4 | 3 | 9 | 5 | 2 | 8 | 4 | 2 |
| Unteres Quartil | 13,5 | 5,5 | 3,5 | 12 | 8 | 5 | 13 | 6 | 5 |
| Median | 15 | 10,5 | 9 | 15 | 12 | 9 | 15 | 11,8 | 9 |
| Oberes Quartil | 17 | 14 | 11 | 17 | 15 | 14 | 17 | 15 | 11 |
| Maximum | 18 | 17 | 14 | 18 | 18 | 18 | 18 | 18 | 18 |
| N (Anzahl) | 12 | 12 | 12 | 10 | 10 | 10 | 22 | 22 | 22 |

Um zu analysieren, ob die Verbesserung des SCORADs bestimmten Parametern zuzuordnen ist oder gleichermaßen von allen Einzelsymptomen getragen wird, wurde Teil B des SCORADs für die einzelnen Parameter: Erytheme, Exkoriation, Ödeme/Papelbildung, Lichenifikation, Nässen/Krustenbildung sowie Trockenheit analysiert.

Die Analyse der Werte für die Erytheme zeigt, dass diese sich in Übereinstimmung mit der Veränderung des Gesamt-SCORADs sowie des Teil B des SCORADs während des Behandlungsverlaufes verbessern (siehe Figur 3 sowie Tabelle 4). Die dokumentierten Verbesserungen stellen sich in der Behandlungsgruppe mit der niedrigeren Dosierung deutlicher dar. Für die Gesamtpopulation sowie die Behandlungsgruppe mit der niedrigeren Dosierung sind die Unterschiede im Vergleich zum Ausgangswert an Tag 0 statistisch signifikant an Tag 7 und an Tag 14.

**Tabelle 4: Ergebnisse der Analyse des Parameters Erythem für beide Behandlungsgruppen sowie die Gesamtpopulation**

| **Erytheme** | **A niedrige Dosis** | | | **B hohe Dosis** | | | **C Gesamtpopulation** | | |
|---|---|---|---|---|---|---|---|---|---|
| Tag | 0 | 7 | 14 | 0 | 7 | 14 | 0 | 7 | 14 |
| Mittelwert | **2,83** | **2,00** | **1,67** | **2,60** | **2,00** | **1,70** | **2,73** | **2,00** | **1,68** |
| Standardabweichung | 0,58 | 0,95 | 0,65 | 0,70 | 0,82 | 0,95 | 0,63 | 0,87 | 0,78 |
| Minimum | 1 | 1 | 1 | 1 | 1 | 0 | 1 | 1 | 0 |
| Unteres Quartil | 3 | 1 | 1 | 2 | 1 | 1 | 2 | 1 | 1 |
| Median | 3 | 2 | 2 | 3 | 2 | 2 | 3 | 2 | 2 |
| Oberes Quartil | 3 | 3 | 2 | 3 | 3 | 2 | 3 | 3 | 2 |
| Maximum | 3 | 3 | 3 | 3 | 3 | 3 | 3 | 3 | 3 |
| N (Anzahl) | 12 | 12 | 12 | 10 | 10 | 10 | 22 | 22 | 22 |

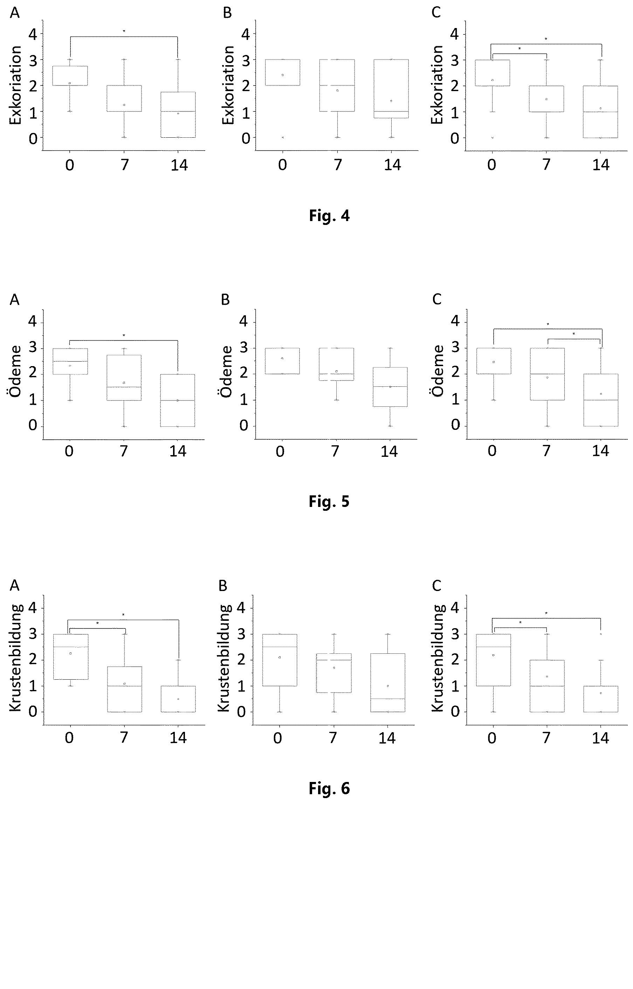

Auch das klinische Symptom Exkoriation verbessert sich über den Verhandlungsverlauf konsistent mit der Veränderung des Gesamt-SCORADs und dem Verlauf des local SCORADs (siehe Figur 4 und Tabelle 5). Signifikante Unterschiede im Vergleich zum Ausgangswert können für die Gesamtpopulation an Tag 7 und an Tag 14, sowie für die Behandlungsgruppe mit der niedrigeren Dosierung dokumentiert werden.

**Tabelle 5: Ergebnisse der Analyse des Parameters Exkoriation für beide Behandlungsgruppen sowie die Gesamtpopulation**

| **Exkoriation** | **A niedrige Dosis** | | | **B hohe Dosis** | | | **C Gesamtpopulation** | | |
|---|---|---|---|---|---|---|---|---|---|
| Tag | 0 | 7 | 14 | 0 | 7 | 14 | 0 | 7 | 14 |
| Mittelwert | **2,08** | **1,25** | **0,92** | **2,40** | **1,80** | **1,40** | **2,23** | **1,50** | **1,14** |
| Standardabweichung | 0,67 | 0,87 | 1,00 | 0,97 | 1,03 | 1,17 | 0,81 | 0,96 | 1,08 |
| Minimum | 1 | 0 | 0 | 0 | 0 | 0 | 0 | 0 | 0 |
| Unteres Quartil | 2 | 1 | 0 | 2 | 1 | 1 | 2 | 1 | 0 |
| Median | 2 | 1 | 1 | 3 | 2 | 1 | 2 | 1 | 1 |
| Oberes Quartil | 2,5 | 2 | 1,5 | 3 | 3 | 3 | 3 | 3 | 2 |
| Maximum | 3 | 3 | 3 | 3 | 3 | 3 | 3 | 3 | 3 |
| N (Anzahl) | 12 | 12 | 12 | 10 | 10 | 10 | 22 | 22 | 22 |

Die Ödem- und Papelbildung reduziert sich ebenfalls im Mittel in beiden Studienpopulationen während des Studienverlaufs (siehe Figur 5 sowie Tabelle 6). Deutlicher sind auch hier die Unterschiede in der Behandlungsgruppe mit der niedrigeren Dosierung. Für die gesamte Studienpopulation ergeben sich signifikante Unterschiede zu beiden weiteren Untersuchungszeitpunkten (Tag 7 und Tag 14) im Vergleich zum Ausgangswert.

**Tabelle 6: Ergebnisse der Analyse des Parameters Ödeme für beide Behandlungsgruppen sowie die Gesamtpopulation**

| **Ödeme** | **A niedrige Dosis** | | | **B hohe Dosis** | | | **C Gesamtpopulation** | | |
|---|---|---|---|---|---|---|---|---|---|
| Tag | 0 | 7 | 14 | 0 | 7 | 14 | 0 | 7 | 14 |
| Mittelwert | **2,33** | **1,67** | **1,00** | **2,60** | **2,10** | **1,50** | **2,45** | **1,86** | **1,23** |
| Standardabweichung | 0,78 | 0,98 | 0,85 | 0,52 | 0,74 | 1,08 | 0,67 | 0,89 | 0,97 |
| Minimum | 1 | 0 | 0 | 2 | 1 | 0 | 1 | 0 | 0 |
| Unteres Quartil | 2 | 1 | 0 | 2 | 2 | 1 | 2 | 1 | 0 |
| Median | 2,5 | 1,5 | 1 | 3 | 2 | 1,5 | 3 | 2 | 1 |
| Oberes Quartil | 3 | 2,5 | 2 | 3 | 3 | 2 | 3 | 3 | 2 |
| Maximum | 3 | 3 | 2 | 3 | 3 | 3 | 3 | 3 | 3 |
| N (Anzahl) | 12 | 12 | 12 | 10 | 10 | 10 | 22 | 22 | 22 |

Die Krustenbildung verbessert sich im Mittel in beiden Behandlungsgruppen während des Studienverlaufs (Figur 6 und Tabelle 7). Besonders deutlich fällt der Unterschied in der Gruppe mit der niedrigeren Dosierung aus. Mit einer mittleren Reduktion auf 0,5 Score-Punkte an Tag 14 ist hier für alle dokumentierten Symptome die deutlichste Veränderung dokumentiert worden. Eine statistische signifikante Verbesserung der Krustenbildung kann für die Behandlungsgruppe mit der niedrigeren Dosierung sowie für die Gesamtpopulation an Tag 7 und an Tag 14 ermittelt werden.

**Tabelle 7: Ergebnisse der Analyse des Parameters Krustenbildung für beide Behandlungsgruppen sowie die Gesamtpopulation**

| **Krustenbildung** | **A niedrige Dosis** | | | **B hohe Dosis** | | | **C Gesamtpopulation** | | |
|---|---|---|---|---|---|---|---|---|---|
| Tag | 0 | 7 | 14 | 0 | 7 | 14 | 0 | 7 | 14 |
| Mittelwert | **2,25** | **1,08** | **0,50** | 2,10 | **1,70** | **1,00** | **2,18** | **1,36** | **0,73** |
| Standardabweichung | 0,87 | 1,08 | 0,67 | 1,10 | 1,06 | 1,25 | 0,96 | 1,09 | 0,98 |
| Minimum | 1 | 0 | 0 | 0 | 0 | 0 | 0 | 0 | 0 |
| Unteres Quartil | 1,5 | 0 | 0 | 1 | 1 | 0 | 1 | 0 | 0 |
| Median | 2,5 | 1 | 0 | 2,5 | 2 | 0,5 | 2,5 | 1 | 0 |
| Oberes Quartil | 3 | 1,5 | 1 | 3 | 2 | 2 | 3 | 2 | 1 |
| Maximum | 3 | 3 | 2 | 3 | 3 | 3 | 3 | 3 | 3 |
| N (Anzahl) | 12 | 12 | 12 | 10 | 10 | 10 | 22 | 22 | 22 |

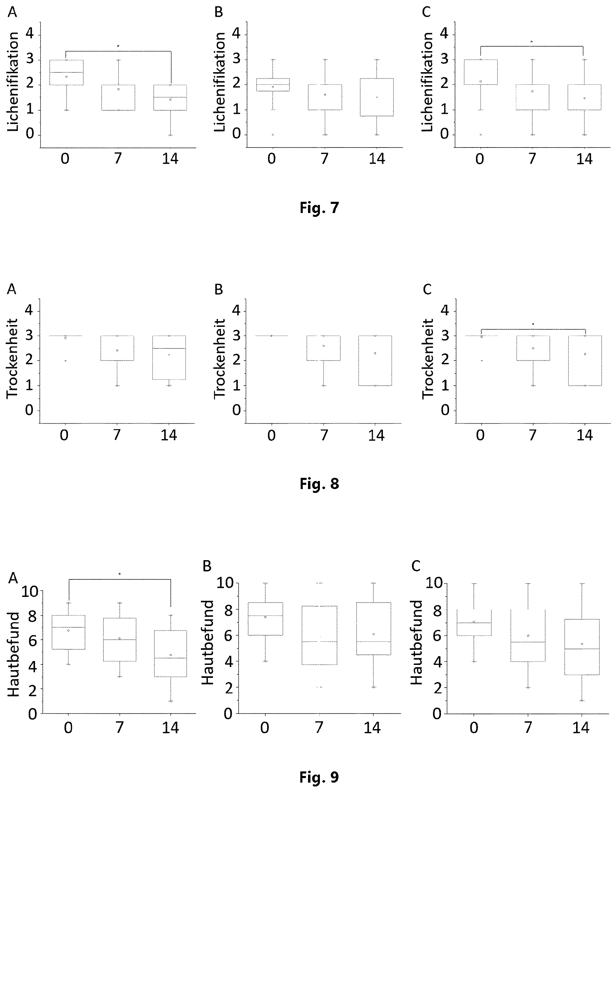

Auch die Anzeichen der Lichenifikation verbessern sich bei den Studienteilnehmern im Mittel tendenziell (Figur 7 und Tabelle 8). Die Unterschiede sind jedoch weniger stark ausgeprägt, als für die bisher beschriebenen Parameter. Die Änderung des Mittelwertes von Tag 0 zu Tag 14 liegt hier unter einem Score-Punkt für die Gesamtpopulation (-0,69), während die korrespondierenden Änderungen für Erytheme, Exkoriation, Ödeme und Krustenbildung jeweils -1,05, -1,09, -1,22 und -1,45 Punkte betrugen. Dennoch erreichen die Unterschiede in der Gesamtpopulation sowie der Behandlungsgruppe mit der niedrigeren Dosierung statistische Signifikanz an Tag 14 im Vergleich zu Tag 0.

**Tabelle 8: Ergebnisse der Analyse des Parameters Lichenifikation für beide Behandlungsgruppen sowie die Gesamtpopulation**

| **Lichenifikation** | **A niedrige Dosis** | | | **B hohe Dosis** | | | **C Gesamtpopulation** | | |
|---|---|---|---|---|---|---|---|---|---|
| | 0 | 7 | 14 | 0 | 7 | 14 | 0 | 7 | 14 |
| Mittelwert | **2,33** | **1,83** | **1,42** | **1,90** | **1,60** | **1,50** | **2,14** | **1,73** | **1,45** |
| Standardabweichung | 0,78 | 0,72 | 0,67 | 0,88 | 0,84 | 1,08 | 0,83 | 0,77 | 0,86 |
| Minimum | 1 | 1 | 0 | 0 | 0 | 0 | 0 | 0 | 0 |
| Unteres Quartil | 2 | 1 | 1 | 2 | 1 | 1 | 2 | 1 | 1 |
| Median | 2,5 | 2 | 1,5 | 2 | 2 | 1,5 | 2 | 2 | 1,5 |
| Oberes Quartil | 3 | 2 | 2 | 2 | 2 | 2 | 3 | 2 | 2 |
| Maximum | 3 | 3 | 2 | 3 | 3 | 3 | 3 | 3 | 3 |
| N (Anzahl) | 12 | 12 | 12 | 10 | 10 | 10 | 22 | 22 | 22 |

Die Mittelwerte des Parameters Hauttrockenheit der Studienteilnehmer verbessern sich im Verlauf der Studie tendenziell (Figur 8 und Tabelle 9). Ähnlich wie bei der Lichenifikation ist hier die Veränderung schwächer als bei den anderen analysierten Parametern. Signifikant ist der Unterschied für die Gesamtpopulation an Tag 14 im Vergleich zum Ausgangwert an Tag 0 (-0,68).

**Tabelle 9: Ergebnisse der Analyse des Parameters Trockenheit für beide Behandlungsgruppen sowie die Gesamtpopulation**

| **Trockenheit** | **A niedrige Dosis** | | | **B hohe Dosis** | | | **C Gesamtpopulation** | | |
|---|---|---|---|---|---|---|---|---|---|
| Tag | 0 | 7 | 14 | 0 | 7 | 14 | 0 | 7 | 14 |
| Mittelwert | **2,92** | **2,42** | **2,25** | **3,00** | **2,60** | **2,30** | **2,95** | **2,50** | **2,27** |
| Standardabweichung | 0,29 | 0,79 | 0,87 | 0,00 | 0,70 | 0,95 | 0,21 | 0,74 | 0,88 |
| Minimum | 2 | 1 | 1 | 3 | 1 | 1 | 2 | 1 | 1 |
| Unteres Quartil | 3 | 2 | 1,5 | 3 | 2 | 1 | 3 | 2 | 1 |
| Median | 3 | 3 | 2,5 | 3 | 3 | 3 | 3 | 3 | 3 |
| Oberes Quartil | 3 | 3 | 3 | 3 | 3 | 3 | 3 | 3 | 3 |
| Maximum | 3 | 3 | 3 | 3 | 3 | 3 | 3 | 3 | 3 |
| N (Anzahl) | 12 | 12 | 12 | 10 | 10 | 10 | 22 | 22 | 22 |

### 6.2 Bewertung durch den Studienteilnehmer

### 6.2.1 Fragebogen zur Symptomatik

Neben der Erhebung des SCORADs durch den Prüfarzt erfolgte die Bewertung der Symptomatik auch durch die Studienteilnehmer selbst. Ihren allgemeinen Hautzustand beurteilten die Teilnehmer dabei im Mittel zu beiden Untersuchungszeitpunkten besser als zum jeweils vorhergehenden Zeitpunkt (Figur 9 und Tabelle 10). Dies gilt für beide Behandlungsgruppen, wenngleich die Unterschiede in der Gruppe mit der niedrigeren Dosierung etwas deutlicher sind. Hier ist der Unterschied an Tag 14 im Vergleich zum Ausgangswert an Tag 0 signifikant. Diese grundsätzliche Tendenz stimmt mit der Bewertung durch den Prüfarzt mittels SCORAD überein.

**Tabelle 10: Bewertung der Hautzustandes durch die Studienteilnehmer: Kennwerte der deskriptiven Statistik für beide Behandlungsgruppen sowie die Gesamtpopulation**

| **Allgemeiner Hautbefund** | **A niedrige Dosis** | | | **B hohe Dosis** | | | **C Gesamtpopulation** | | |
|---|---|---|---|---|---|---|---|---|---|
| Tag | **0** | **7** | **14** | **0** | **7** | **14** | **0** | **7** | **14** |
| Mittelwert | 6,75 | 6,08 | 4,75 | 7,40 | 5,90 | 6,10 | 7,05 | 6,00 | 5,36 |
| Standardabweichung | 1,54 | 2,02 | 2,30 | 1,84 | 2,60 | 2,69 | 1,68 | 2,25 | 2,52 |
| Minimum | 4 | 3 | 1 | 4 | 2 | 2 | 4 | 2 | 1 |
| Unteres Quartil | 5,5 | 4,5 | 3 | 6 | 4 | 5 | 6 | 4 | 3 |
| Median | 7 | 6 | 4,5 | 7,5 | 5,5 | 5,5 | 7 | 5,5 | 5 |
| Oberes Quartil | 8 | 7,5 | 6,5 | 8 | 8 | 8 | 8 | 8 | 7 |
| Maximum | 9 | 9 | 8 | 10 | 10 | 10 | 10 | 10 | 10 |
| N (Anzahl) | 12 | 12 | 12 | 10 | 10 | 10 | 22 | 22 | 22 |

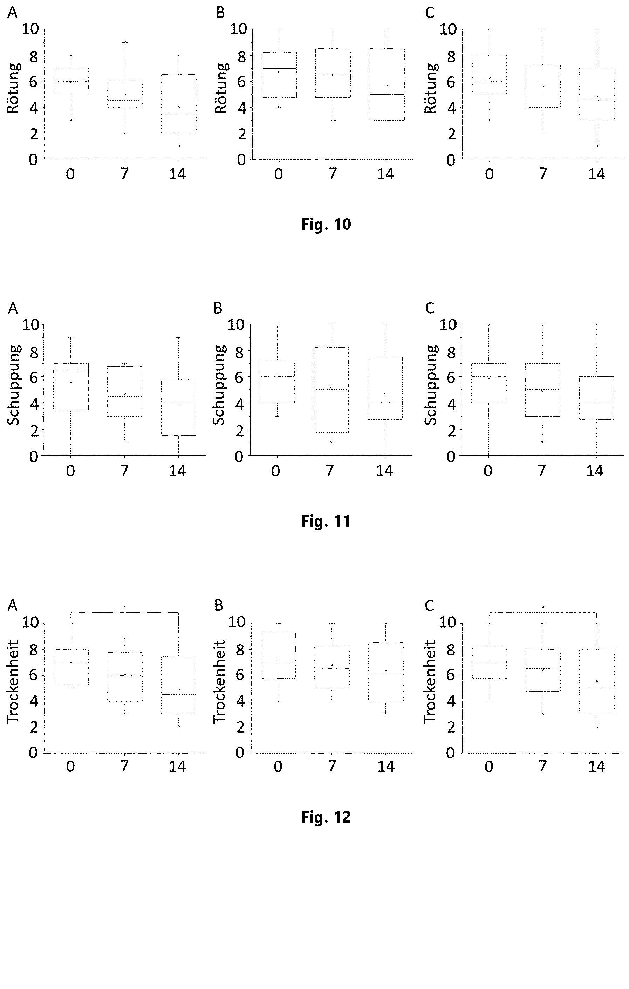

Die Bewertung der Rötung der behandelten Hautstellen durch die Studienteilnehmer ist vergleichbar mit der Bewertung des gesamten Hautzustandes: Es besteht eine Tendenz zur Verbesserung der Rötung in beiden Behandlungsgruppen sowie demzufolge in der Gesamtpopulation (Figur 10 und Tabelle 11). Eine statistische Signifikanz kann jedoch nicht erreicht werden - im Gegensatz zu der Bewertung der Erytheme durch den Prüfarzt im Rahmen des SCORADs.

**Tabelle 11: Bewertung der Rötung der betroffenen Hautstellen durch die Studienteilnehmer: Kennwerte der deskriptiven Statistik für beide Behandlungsgruppen sowie die Gesamtpopulation**

| **Rötung** | **A niedrige Dosis** | | | **B hohe Dosis** | | | **C Gesamtpopulation** | | |
|---|---|---|---|---|---|---|---|---|---|
| Tag | **0** | **7** | **14** | **0** | **7** | **14** | **0** | **7** | **14** |
| Mittelwert | **5,92** | **4,92** | **4,00** | **6,70** | **6,50** | **5,70** | **6,27** | **5,64** | **4,77** |
| Standardabweichung | 1,51 | 2,11 | 2,41 | 2,11 | 2,37 | 2,87 | 1,80 | 2,32 | 2,71 |
| Minimum | 3 | 2 | 1 | 4 | 3 | 3 | 3 | 2 | 1 |
| Unteres Quartil | 5 | 4 | 2 | 5 | 5 | 3 | 5 | 4 | 3 |
| Median | 6 | 4,5 | 3,5 | 7 | 6,5 | 5 | 6 | 5 | 4,5 |
| Oberes Quartil | 7 | 6 | 6 | 8 | 8 | 8 | 8 | 7 | 7 |
| Maximum | 8 | 9 | 8 | 10 | 10 | 10 | 10 | 10 | 10 |
| N (Anzahl) | 12 | 12 | 12 | 10 | 10 | 10 | 22 | 22 | 22 |

Auch die Bewertung der Schuppung der behandelten Hautstellen durch die Studienteilnehmer zeigt im Mittel eine tendenzielle Verbesserung dieser Symptomatik (Figur 11 und Tabelle 12) in Übereinstimmung mit der Verbesserung des Hautzustandes laut des Teilnehmerfragebogens und des SCORADs. Auch hier wird keine statistische Signifikanz erreicht.

**Tabelle 12: Bewertung der Schuppung der betroffenen Hautstellen durch die Studienteilnehmer: Kennwerte der deskriptiven Statistik für beide Behandlungsgruppen sowie die Gesamtpopulation**

| **Schuppung** | **A niedrige Dosis** | | | **B hohe Dosis** | | | **C Gesamtpopulation** | | |
|---|---|---|---|---|---|---|---|---|---|
| Tag | **0** | **7** | **14** | **0** | **7** | **14** | **0** | **7** | **14** |
| Mittelwert | **5,58** | **4,67** | **3,83** | **6,00** | **5,20** | **4,60** | **5,77** | **4,91** | **4,18** |
| Standardabweichung | 2,54 | 1,97 | 2,52 | 2,11 | 3,26 | 3,13 | 2,31 | 2,58 | 2,77 |
| Minimum | 0 | 1 | 0 | 3 | 1 | 0 | 0 | 1 | 0 |
| Unteres Quartil | 4 | 3 | 2 | 4 | 2 | 3 | 4 | 3 | 3 |
| Median | 6,5 | 4,5 | 4 | 6 | 5 | 4 | 6 | 5 | 4 |
| Oberes Quartil | 7 | 6,5 | 5,5 | 7 | 8 | 7 | 7 | 7 | 6 |
| Maximum | 9 | 7 | 9 | 10 | 10 | 10 | 10 | 10 | 10 |
| N (Anzahl) | 12 | 12 | 12 | 10 | 10 | 10 | 22 | 22 | 22 |

Im Gegensatz zu den Ergebnissen der Arztbewertung wurde die Verbesserung der Hauttrockenheit durch den Patienten selbst als deutlicher empfunden und bewertet (Figur 12 und Tabelle 13). In der Behandlungsgruppe mit der niedrigeren Dosierung waren die Unterschiede signifikant im Vergleich zu Tag 0, ebenso wir in der gesamten Studienpopulation.

**Tabelle 13: Bewertung der Trockenheit der betroffenen Hautstellen durch die Studienteilnehmer: Kennwerte der deskriptiven Statistik für beide Behandlungsgruppen sowie die Gesamtpopulation**

| **Trockenheit** | **A niedrige Dosis** | | | **B hohe Dosis** | | | **C Gesamtpopulation** | | |
|---|---|---|---|---|---|---|---|---|---|
| Tag | **0** | **7** | **14** | **0** | **7** | **14** | **0** | **7** | **14** |
| Mittelwert | **7,00** | **6,00** | **4,92** | **7,30** | **6,80** | **6,30** | **7,14** | **6,36** | **5,55** |
| Standardabweichung | 1,65 | 2,09 | 2,35 | 2,00 | 1,93 | 2,50 | 1,78 | 2,01 | 2,46 |
| Minimum | 5 | 3 | 2 | 4 | 4 | 3 | 4 | 3 | 2 |
| Unteres Quartil | 5,5 | 4 | 3 | 6 | 5 | 4 | 6 | 5 | 3 |
| Median | 7 | 6 | 4,5 | 7 | 6,5 | 6 | 7 | 6,5 | 5 |
| Oberes Quartil | 8 | 7,5 | 7 | 9 | 8 | 8 | 8 | 8 | 8 |
| Maximum | 10 | 9 | 9 | 10 | 10 | 10 | 10 | 10 | 10 |
| N (Anzahl) | 12 | 12 | 12 | 10 | 10 | 10 | 22 | 22 | 22 |

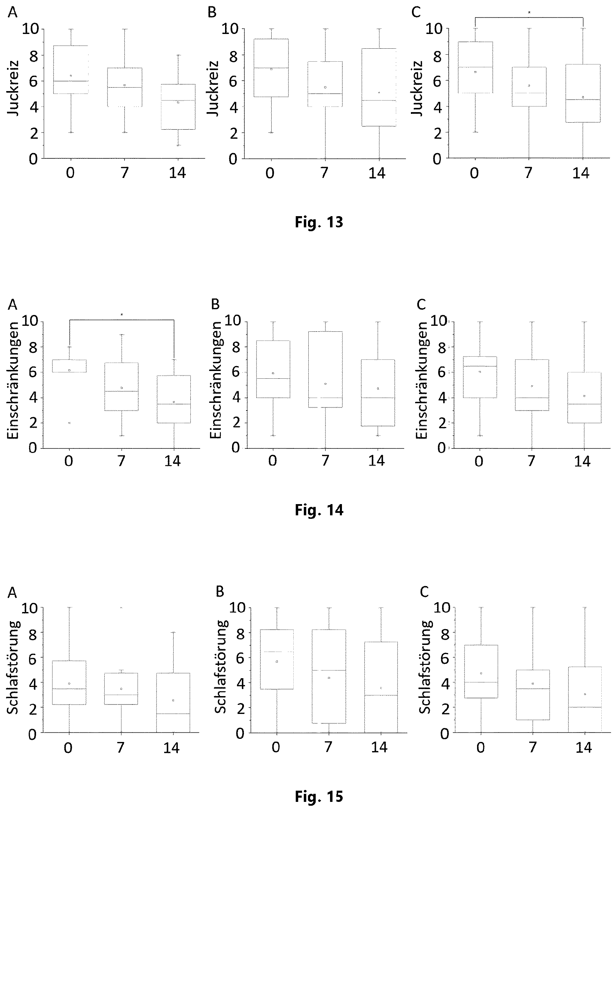

Der Juckreiz konnte im Mittel gemäß der Teilnehmerbewertung im Studienverlauf gemildert werden (Figur 13 und Tabelle 14). Für die Gesamtpopulation war der Unterschied statistisch signifikant im Vergleich zum Ausgangswert.

**Tabelle 14: Bewertung des Juckreizes der betroffenen Hautstellen durch die Studienteilnehmer: Kennwerte der deskriptiven Statistik für beide Behandlungsgruppen sowie die Gesamtpopulation**

| **Juckreiz** | **A niedrige Dosis** | | | **B hohe Dosis** | | | **C Gesamtpopulation** | | |
|---|---|---|---|---|---|---|---|---|---|
| Tag | 0 | **7** | **14** | **0** | **7** | **14** | **0** | **7** | **14** |
| Mittelwert | **6,42** | **5,67** | **4,33** | **6,90** | **5,50** | **5,10** | **6,64** | **5,59** | **4,68** |
| Standardabweichung | 2,27 | 2,42 | 2,27 | 2,60 | 2,80 | 3,54 | 2,38 | 2,54 | 2,87 |
| Minimum | 2 | 2 | 1 | 2 | 0 | 0 | 2 | 0 | 0 |
| Unteres Quartil | 5 | 4 | 2,5 | 5 | 4 | 3 | 5 | 4 | 3 |
| Median | 6 | 5,5 | 4,5 | 7 | 5 | 4,5 | 7 | 5 | 4,5 |
| Oberes Quartil | 8,5 | 7 | 5,5 | 9 | 7 | 8 | 9 | 7 | 7 |
| Maximum | 10 | 10 | 8 | 10 | 10 | 10 | 10 | 10 | 10 |
| N (Anzahl) | 12 | 12 | 12 | 10 | 10 | 10 | 22 | 22 | 22 |

Auch die Lebensqualität der Studienteilnehmer wurde anhand des Fragebogens erfasst. Hier wurden die Einschränkungen im Alltag (Figur 14 und Tabelle 15) sowie durch die Hauterkrankung verursachte Schlafstörungen dokumentiert (Figur 15 und Tabelle 16).

Beide Parameter verbesserten sich im Studienverlauf tendenziell. Ein statistisch signifikanter Unterschied kann jedoch nur für die Einschränkungen im Alltag für die Teilnehmergruppe mit der höheren Dosierung an Tag 14 im Vergleich zu Tag 0 festgestellt werden.

**Tabelle 15: Bewertung des Einschränkungen im Alltag der betroffenen Hautstellen durch die Studienteilnehmer: Kennwerte der deskriptiven Statistik für beide Behandlungsgruppen sowie die Gesamtpopulation**

| **Einschränkungen im Alltag** | **A niedrige Dosis** | | | **B hohe Dosis** | | | **C Gesamtpopulation** | | |
|---|---|---|---|---|---|---|---|---|---|
| Tag | **0** | **7** | **14** | **0** | **7** | **14** | **0** | **7** | **14** |
| Mittelwert | **6,17** | **4,75** | **3,67** | **5,90** | **5,10** | **4,70** | **6,05** | **4,91** | **4,14** |
| Standardabweichung | 1,85 | 2,34 | 2,19 | 2,88 | 3,51 | 3,34 | 2,32 | 2,86 | 2,75 |
| Minimum | 2 | 1 | 0 | 1 | 0 | 1 | 1 | 0 | 0 |
| Unteres Quartil | 6 | 3 | 2 | 4 | 4 | 2 | 4 | 3 | 2 |
| Median | 7 | 4,5 | 3,5 | 5,5 | 4 | 4 | 6,5 | 4 | 3,5 |
| Oberes Quartil | 7 | 6,5 | 5,5 | 8 | 9 | 6 | 7 | 7 | 6 |
| Maximum | 8 | 9 | 7 | 10 | 10 | 10 | 10 | 10 | 10 |
| N (Anzahl) | 12 | 12 | 12 | 10 | 10 | 10 | 22 | 22 | 22 |

**Tabelle 16: Bewertung der durch die Hauterkrankung bedingten Schlafstörungen durch die Studienteilnehmer: Kennwerte der deskriptiven Statistik für beide Behandlungsgruppen sowie die Gesamtpopulation**

| **Schlafstörungen** | **A niedrige Dosis** | | | **B hohe Dosis** | | | **C Gesamtpopulation** | | |
|---|---|---|---|---|---|---|---|---|---|
| Tag | **0** | **7** | **14** | **0** | **7** | **14** | **0** | **7** | **14** |
| Mittelwert | **3,92** | **3,50** | **2,58** | **5,70** | **4,40** | **3,60** | **4,73** | **3,91** | **3,05** |
| Standardabweichung | 2,84 | 2,61 | 2,75 | 3,16 | 3,78 | 3,78 | 3,06 | 3,15 | 3,21 |
| Minimum | 0 | 0 | 0 | 0 | 0 | 0 | 0 | 0 | 0 |
| Unteres Quartil | 2,5 | 2,5 | 0 | 4 | 1 | 0 | 3 | 1 | 0 |
| Median | 3,5 | 3 | 1,5 | 6,5 | 5 | 3 | 4 | 3,5 | 2 |
| Oberes Quartil | 5,5 | 4,5 | 4,5 | 8 | 8 | 7 | 7 | 5 | 5 |
| Maximum | 10 | 10 | 8 | 10 | 10 | 10 | 10 | 10 | 10 |
| N (Anzahl) | 12 | 12 | 12 | 10 | 10 | 10 | 22 | 22 | 22 |

### 6.3 Analyse der Subgruppe Kinder

Da die Prävalenz der Neurodermitis im Kindesalter eine deutlich höher liegt als im Erwachsenenalter, wurde die Wirksamkeit des Testproduktes für die Subgruppe der Kinder (5 bis 11 Jahre) zusätzlich ausgewertet. Die Ergebnisse zeigen, dass das probiotische Bad in dieser Altersgruppe eine vergleichbar gute Wirksamkeit zeigt, wie in der Gesamtpopulation.

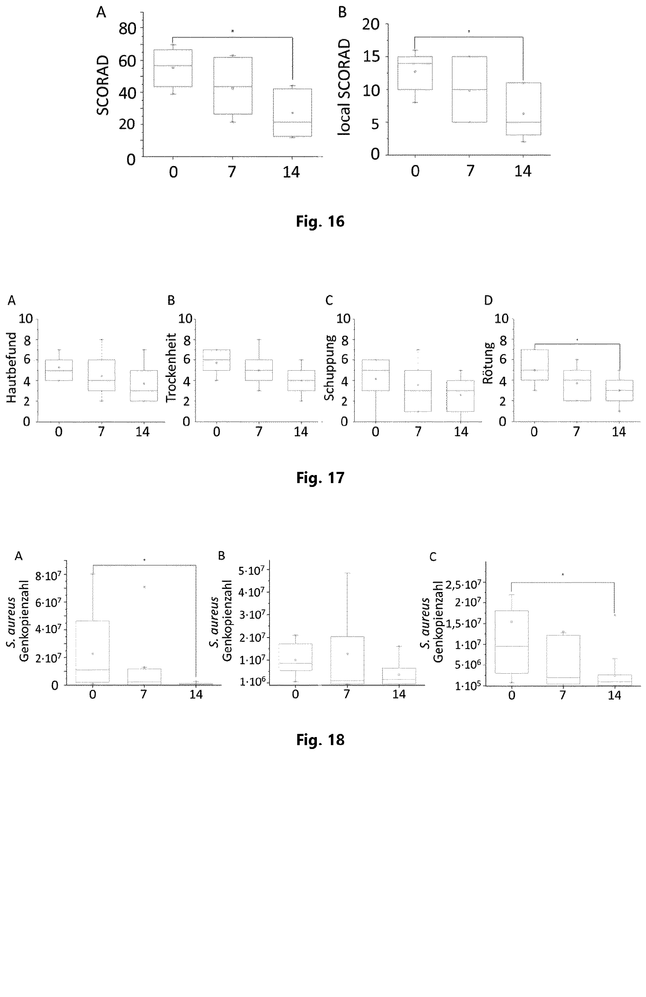

SCORAD sowie localSCORAD können zu beiden Untersuchungszeitpunkten jeweils deutlich zum vorhergehenden Zeitpunkt gesenkt werden (Abbildung 16 und Tabelle 17). Die Unterschiede sind jeweils signifikant an Tag 14 im Vergleich zum Ausgangswert an Tag 0, obwohl die Subgruppe nur eine geringe Teilnehmerzahl (n=7) umfasste.

**Tabelle 17: Ergebnisse der Analyse des SCORADs und des local SCORADs der Subgruppe Kinder**

| | **A SCORAD** | | | **B local SCORAD** | | |
|---|---|---|---|---|---|---|
| Tag | 0 | 7 | 14 | 0 | 7 | 14 |
| Mittelwert | **55,39** | **42,63** | **27,16** | **12,71** | **9,86** | **6,29** |
| Standardabweichung | 11,25 | 15,96 | 14,60 | 2,87 | 4,18 | 3,95 |
| Minimum | 38,90 | 21,40 | 11,80 | 8 | 5 | 2 |
| Unteres Quartil | 43,60 | 26,40 | 12,40 | 10 | 5 | 3 |
| Median | 56,90 | 43,60 | 21,50 | 14 | 10 | 5 |
| Oberes Quartil | 66,80 | 61,90 | 42,30 | 15 | 15 | 11 |
| Maximum | 69,80 | 63,00 | 44,30 | 16 | 15 | 11 |
| N (Anzahl) | 7 | 7 | 7 | 7 | 7 | 7 |

Auch die Auswertung der Teilnehmerfragebögen zeigte in der Subgruppe der Kinder eine Verbesserung der Hauttrockenheit, Schuppung und Rötung der Haut während des Studienverlaufs. Demzufolge wurde auch der gesamte Hautbefund zu beiden Folgeuntersuchungen im Mittel jeweils besser bewertet als zum vorhergehenden Zeitpunkt (Abbildung 17 und Tabelle 18).

**Tabelle 18: Bewertung des allgemeinen Hautzustandes (A), der Trockenheit (B), der Schuppung (C) sowie der Rötung (D) durch die Studienteilnehmer. Kennwerte der deskriptiven Statistik für die Subgruppe Kinder.**

| | **A Hautbefund** | | | **B Trockenheit** | | |
|---|---|---|---|---|---|---|
| Tag | 0 | 7 | 14 | 0 | 7 | 14 |
| Mittelwert | **5,29** | **4,43** | **3,71** | **5,71** | **5,00** | **4,00** |
| Standardabweichung | 1,11 | 1,99 | 1,80 | 1,11 | 1,63 | 1,41 |
| Minimum | 4 | 2 | 2 | 4 | 3 | 2 |
| Unteres Quartil | 4 | 3 | 2 | 5 | 4 | 3 |
| Median | 5 | 4 | 3 | 6 | 5 | 4 |
| Oberes Quartil | 6 | 6 | 5 | 7 | 6 | 5 |
| Maximum | 7 | 8 | 7 | 7 | 8 | 6 |
| N (Anzahl) | 7 | 7 | 7 | 7 | 7 | 7 |

| | **C Schuppung** | | | **D Rötung** | | |
|---|---|---|---|---|---|---|
| Mittelwert | **4,14** | **3,57** | **2,57** | **5,00** | **3,71** | **3,00** |
| Standardabweichung | 2,12 | 2,23 | 1,90 | 1,53 | 1,50 | 1,29 |
| Minimum | 0 | 1 | 0 | 3 | 2 | 1 |
| Unteres Quartil | 3 | 1 | 1 | 4 | 2 | 2 |
| Median | 5 | 3 | 3 | 5 | 4 | 3 |
| Oberes Quartil | 6 | 5 | 4 | 7 | 5 | 4 |
| Maximum | 6 | 7 | 5 | 7 | 6 | 5 |
| N (Anzahl) | 7 | 7 | 7 | 7 | 7 | 7 |
| Maximum | 6 | 7 | 5 | 7 | 6 | 5 |
| N (Anzahl) | 7 | 7 | 7 | 7 | 7 | 7 |

Auch die Parameter zur Lebensqualität wie Einschränkungen im Alltag und Schlafstörungen konnten während der Studie verbessert werden (Abbildung 18 und Tabelle 19). Signifikante Unterschiede wurden jedoch nicht erreicht.

**Tabelle 19: : Bewertung der Einschränkungen im Alltag (A), des Juckreizes (B) und der durch die Hauterkrankung bedingten Einschlafstörungen (C) durch die Studienteilnehmer. Kennwerte der deskriptiven Statistik.**

| | **A Einschränkungen im Alltag** | | | **B Juckreiz** | | | **C Schlafstörungen** | | |
|---|---|---|---|---|---|---|---|---|---|
| Tag | **0** | **7** | **14** | **0** | **7** | **14** | **0** | **7** | **14** |
| Mittelwert | **4,43** | **2,86** | **2,57** | **5,29** | **4,57** | **3,00** | **3,71** | **2,86** | **1,29** |
| Standardabweichung | 1,72 | 1,46 | 1,51 | 1,89 | 1,51 | 1,73 | 2,63 | 1,95 | 2,21 |
| Minimum | 2 | 1 | 0 | 2 | 2 | 1 | 0 | 0 | 0 |
| Unteres Quartil | 3 | 1 | 2 | 4 | 4 | 1 | 2 | 1 | 0 |
| Median | 4 | 4 | 3 | 5 | 5 | 3 | 4 | 3 | 0 |
| Oberes Quartil | 6 | 4 | 3 | 7 | 5 | 5 | 7 | 5 | 4 |
| Maximum | 7 | 4 | 5 | 7 | 7 | 5 | 7 | 5 | 5 |
| N (Anzahl) | 7 | 7 | 7 | 7 | 7 | 7 | 7 | 7 | 7 |

### 6.4 Mikrobiologische Untersuchungen

Von den Teilnehmern, die die Studie vollständig abgeschlossen haben, wurden an Tag 0, Tag 7 und Tag 14 Abstriche an Hautpartien mit atopischem Ekzem genommen. Von diesen Hautabstrichen wurden die Nukleinsäuren extrahiert (siehe 4.1 und 4.2) und mittels quantitativer PCR wurde die Genkopienzahl an S. aureus (4.3) aus dem jeweiligen Nukleinsäureextrakt bestimmt. Dieselben Nukleinsäureextrakte wurden zudem für eine bakterielle 16s rRNA Gen und pilzliche ITS Sequenzierung (siehe 4.4) genutzt. Einige Extrakte zeigten nur eine ungenügende Quantität an Nukleinsäuren. Diese ungenügende Quantität an Nukleinsäuren führte dazu, dass diese Extrakte eine äußerst geringe Genkopienzahl an S. aureus aufwiesen und in der Sequenzierung nur eine geringe Anzahl an Sequenzen gewonnen werden konnten. Daher wurden die Probe des Probanden 15 am Tag 7 für die Charakterisierung der bakteriellen Gemeinschaft und die Probe des Probanden 6 (Tag 7, 68 Sequenz reads), 14 (Tag 0, 16 16 Sequenz reads), Probanden 16 (Tag 14, 1 Sequenz reads) und Probanden 27 (Tag 0 und 7, 6 und 0 Sequenz reads) für die Quantifizierung der Genkopienzahl an S. aureus und der Charakterisierung der pilzlichen Gemeinschaft nicht genutzt.

### 6.4.1 Quantifizierung der Genkopienzahl an S. aureus

Die Auswertung der Quantifizierung des Genkopienzahl an S. aureus zeigte, dass in beiden Behandlungsgruppen ein deutlicher Trend zur Verringerungen der Genkopienzahl von S.aureus bestand (siehe Figur 18 und Tabelle 20). Sowohl an Tag 7 als auch an Tag 14 reduzierte sich der Mittelwert der Genkopienzahl im Vergleich zum vorhergehenden Zeitpunkt. In der Behandlungsgruppe mit der niedrigeren Dosierung und der Gesamtpopulation stellte sich dieser Trend etwas deutlicher dar; im Vergleich zum Ausgangswert an Tag 0 war der Unterschied in der Genkopienzahl hier an Tag 14 statistisch signifikant.

**Tabelle 20: Quantifizierung der Genkopienzahl von S. aureus: Kennwerte der deskriptiven Statistik für beide Behandlungsgruppen sowie für die Gesamtpopulation.**

| ***S. aureus* Genkopienzahl** | **A niedrige Dosis** | | | **B hohe Dosis** | | | **C Gesamtpopulation** | | |
|---|---|---|---|---|---|---|---|---|---|
| Tag | **0** | **7** | **14** | **0** | **7** | **14** | **0** | **7** | **14** |
| Mittelwert | 22580400 | 12641800 | 863744 | 11104600 | 13725600 | 4621598 | 15546000 | 13134500 | 2571859 |
| Standardabweichung | 26201200 | 22600700 | 1008075 | 6512114 | 23468500 | 5465113 | 19750800 | 22447900 | 4123173 |
| Minimum | 848083 | 23030 | 0 | 1476833 | 356683 | 38948 | 848083 | 23030 | 0 |
| Unteres Quartil | 2524250 | 297475 | 48428 | 6475000 | 611917 | 531767 | 3416250 | 513717 | 183600 |
| Median | 11034500 | 2322917 | 437808 | 9789742 | 2019167 | 2403750 | 9617211 | 2113583 | 1078833 |
| Oberes Quartil | 44979600 | 10671700 | 1263000 | 17926200 | 12036700 | 6620000 | 17926200 | 12036700 | 2763833 |
| Maximum | 80200000 | 70850000 | 2773500 | 22062500 | 65066700 | 17136700 | 80200000 | 70850000 | 17136700 |
| N (Anzahl) | 12 | 12 | 12 | 10 | 10 | 10 | 22 | 22 | 22 |

### 6.4.2 Quantifizierung der Genkopienzahl an S. aureus in der Subgruppe Kinder

Die Subgruppe der Kinder umfasste nur wenige Studienteilnehmer: In der Behandlungsgruppe mit der niedrigeren Dosierung waren 4 Kinder eingeschlossen, in der Behandlungsgruppe mit der hohen Dosierung 3 Kinder. (siehe auch 6.3).

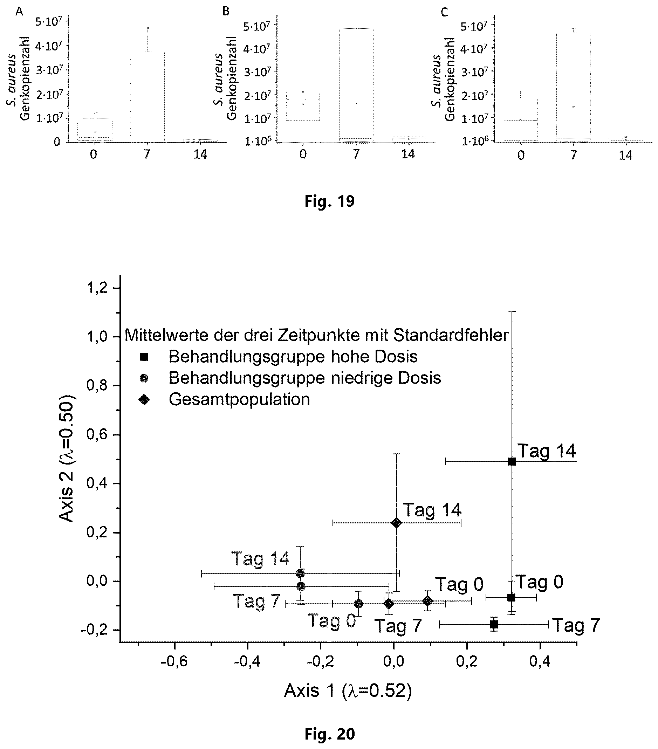

Die Genkopienzahl von S. aureus nahm in der Behandlungsgruppe mit der höheren Dosierung und in der Gesamtpopulation über die Zeit ab, während in der Behandlungsgruppe mit der niedrigeren Dosierung erst an 14 Tag eine Abnahme der Genkopienzahl festgestellt werden konnte (Figur 19). Die Unterschiede in der Genkopienzahl erreichten in keiner Behandlungsgruppe statistische Signifikanz über die Behandlungsdauer.

**Tabelle 21: Quantifizierung der Genkopienzahl von S. aureus: Kennwerte der deskriptiven Statistik für beide Behandlungsgruppen sowie die Gesamtpopulation der Subgruppe Kinder.**

| ***S. aureus* Genkopienzahl Kinder** | **A niedrige Dosis** | | | **B hohe Dosis** | | | **C Gesamtpopulation** | | |
|---|---|---|---|---|---|---|---|---|---|
| Tag | **0** | **7** | **14** | **0** | **7** | **14** | **0** | **7** | **14** |
| Mittelwert | 4428012 | 14041700 | 627383 | 16880400 | 17277200 | 1660033 | 9764745 | 15428300 | 1069948 |
| Standardabweichung | 5485269 | 22450400 | 711271 | 6470125 | 27803900 | 1304185 | 8561635 | 22642600 | 1060469 |
| Minimum | 848083 | 23030 | 0 | 9628667 | 513717 | 183600 | 848083 | 23030 | 0 |
| Unteres Quartil | 916233 | 247515 | 25600 | 9628667 | 513717 | 183600 | 984383 | 472000 | 51200 |
| Median | 2200317 | 4466000 | 548100 | 18950000 | 1946167 | 2141333 | 9628667 | 1946167 | 1045000 |
| Oberes Quartil | 7939792 | 27835800 | 1229167 | 22062500 | 49371700 | 2655167 | 18950000 | 47211700 | 2141333 |
| Maximum | 12463300 | 1413333 | 47211700 | 22062500 | 49371700 | 2655167 | 22062500 | 49371700 | 2655167 |
| N (Anzahl) | 4 | 4 | 4 | 3 | 3 | 3 | 7 | 7 | 7 |

### 6.4.3 Charakterisierung des bakteriellen Hautmikrobioms

Anhand der Berechnung der ökologischen Indizes konnte kein signifikanter Unterschied der α-Diversität zwischen den beiden Behandlungsgruppen festgestellt werden (siehe Tabelle 22). Die bakterielle Diversität (Richness) war in der Behandlungsgruppe mit der höheren Dosis im Vergleich zu der geringen Dosis zu Beginn der Studie leicht erhöht. Die bakterielle Diversität (Richness) nahm in beiden Gruppen und in der Gesamtpopulation über die Behandlungsdauer zu. Alle anderen Indizes zeigten keine ausgeprägten Unterschiede zwischen den Behandlungsgruppen und über die Zeit.

**Tabelle 22: Mittelwerte der ökologischen Indizes Richness, Shannon-Index, Simpson-Index und Pielou's Evenness mit Standardabweichung (SD) und Standardfehler (SE) für beide Behandlungsgruppen sowie die Gesamtpopulation. Die Indices wurden mit den Packages microbiome und phyloseq berechnet. Mittels ANOVA wurde bestimmt, ob ein signifikanter Unterschied zwischen den Behandlungsgruppen und der Anwendungszeit bestanden hatte.**

| **Behandlungsgruppe** | **Richness** | **Shannon** | **Simpson** | **Eveness** | **SD** | **SE** |
|---|---|---|---|---|---|---|
| **Zeitpunkt Tag 0** | | | | | | |
| **Hohe Dosis** | 130 | 2,15 | 0,62 | 0,45 | 152,19 | 14,96 |
| **niedrige Dosis** | 109 | 2,18 | 0,64 | 0,48 | 131,01 | 13,91 |
| **Gesamtpopulation** | 119 | 2,17 | 0,63 | 0,47 | 140,64 | 14,39 |

| **Zeitpunkt Tag 7** | | | | | | |
|---|---|---|---|---|---|---|
| **Hohe Dosis** | 160 | 1,96 | 0,55 | 0,40 | 182,67 | 12,84 |
| **niedrige Dosis** | 151 | 2,27 | 0,63 | 0,45 | 171,20 | 12,12 |
| **Gesamtpopulation** | 155 | 2,13 | 0,60 | 0,43 | 176,12 | 12,43 |

| **Zeitpunkt Tag 14** | | | | | | |
|---|---|---|---|---|---|---|
| **Hohe Dosis** | 157 | 2,43 | 0,68 | 0,49 | 182,45 | 15,12 |
| **niedrige Dosis** | 148 | 2,23 | 0,63 | 0,46 | 170,50 | 14,27 |
| **Gesamtpopulation** | 152 | 2,32 | 0,66 | 0,48 | 175,93 | 14,65 |

Die Zusammensetzung der bakteriellen Gemeinschaftsstruktur änderte sich in beiden Behandlungsgruppen sowie der Gesamtpopulation nicht signifikant über die Zeit (Figur 20). Es war jedoch ein Unterschied der zeitlichen Entwicklung der Zusammensetzung der bakteriellen Gemeinschaftsstruktur zwischen den beiden Behandlungsgruppen mit unterschiedlicher Dosierung zu verzeichnen (Figur 20).

Die relative Abundanz und die phylogenetische Zusammensetzung der bakteriellen Gemeinschaftsstruktur änderten sich über die Zeit. In beiden Behandlungsgruppen sowie der Gesamtpopulation nahmen die Anteile der Gattungen Lactobacillus und Bifidobacterium insbesondere im Zeitraum der ersten 7 Behandlungstage zu. Bis zum Untersuchungszeitpunkt an Tag 14 schienen sich die Anteile dann auf diesem höheren Niveau zu stabilisieren. Die Gattung Streptokokkus nahm tendenziell ebenfalls zu. In der Behandlungsgruppe der niedrigen Dosierung lagen die Anteile jedoch an Tag 14 unter den Ausgangswerten an Tag 0. Vertreter aller drei Gattungen sind Bestandteil des probiotischen Testproduktes.

Weiterhin nahmen über die Behandlungsdauer auch die relativen Abundanzen der Familie der Corynebakterien und einige Familien der Abteilung der Proteobakterien im Vergleich zum Tag 0 zu, obwohl diese nicht Bestandteil des Bades waren. Zeitgleich sank die Sequenzreads-Häufigkeit der Gattung Staphylococcus über die Behandlungsdauer, wenngleich diese Abnahme nicht signifikant war.

### 7. Diskussion der Ergebnisse

### 7.1 Klinische Wirksamkeitsparameter

Sowohl die Auswertung des SCORADs als auch der Teilnehmerfragebögen zeigen, dass im Mittel für alle Parameter eine Verbesserung eintrat. Dies gilt für beide Dosierungen des Testproduktes, die sich hinsichtlich der im Rahmen dieser Studie ermittelten Wirksamkeit nur geringfügig unterschieden. Es ist davon auszugehen, dass eine leichte Tendenz zugunsten der niedrigeren Dosierung aufgrund der geringen Teilnehmerzahl pro Gruppe zufällig entstanden ist. Die niedrigere Dosierung des probiotischen Bades reicht diesen Ergebnissen zufolge also aus, um eine klinische Verbesserung der Symptomatik zu erzielen.

Die Auswertung des SCORADs sowie des sogenannten local SCORADs (Teil B des SCORADs) ergaben eine signifikante Verbesserung der klinischen Symptomatik während des Studienverlaufes. Eine Teilauswertung der einzelnen Parameter des SCORADs (Teil B) zeigen, dass diese Veränderung von allen Parametern getragen wird und es kein Einzelsymptom gibt, welches während des Studienverlaufes völlig unverändert geblieben ist oder sich verschlechtert hat. Dabei verbesserten sich jedoch die Parameter Lichenifikation und Hauttrockenheit innerhalb der 14-tägigen Anwendungsdauer weniger deutlich als die Symptome Erythem, Exkoriation, und Krustenbildung. Während Letztere eher Anzeichen eines akuten Entzündungsgeschehens sind und demzufolge schneller abklingen, stellt Lichenifikation eine Veränderung der Hautstruktur dar. Diese verändert sich langsamer und es erfordert mehrere Wochen bis Monate, bis sich die lederartigen Veränderungen zurückbilden. Unter diesem Aspekt kann die leichte Tendenz zur Verbesserung der Lichenifikation unter Behandlung mit dem Testprodukt als erste Phase der Rückbildung interpretiert werden.

Der im Rahmen des SCORADs dokumentierte geringere Einfluss des probiotischen Bades auf die Hauttrockenheit, kann der Darreichungsform "Bad" als solches geschuldet sein, insbesondere im Hinblick auf die tägliche Anwendung. Es ist jedoch zu berücksichtigen, dass sich die Trockenheit insgesamt verbesserte und nicht verschlechterte. Darüber hinaus weicht in diesem Fall die Bewertung des Arztes von der Bewertung der Studienteilnehmer ab, welche die Veränderung der Hauttrockenheit besser beurteilten.

Insgesamt bestätigen die Ergebnisse des Teilnehmerfragebogens die Verbesserung der Symptomatik die durch den Arzt mittels SCORAD dokumentiert wurde. Auch hier lässt sich für alle Parameter: Rötung, Schuppung, Trockenheit, Juckreiz und allgemeiner Hautzustand eine tendenzielle, z.T. signifikante Verbesserung feststellen.

Dabei wird die Linderung der Symptome - mit Ausnahme der Hauttrockenheit - durch die Studienteilnehmer jedoch meist etwas weniger deutlich bewertet als durch den Arzt. Hier kann eine subjektive Skepsis gegenüber der neuen Anwendungsform eine Rolle spielen. Es ist weiterhin zu berücksichtigen, dass sieben Tage vor dem Start der Anwendung alle wirkstoffhaltigen topischen Präparate abgesetzt wurden und der Patient somit möglicherweise eine Verschlechterung seiner Symptomatik erwartete.

Dennoch zeigt auch die Bewertung der Lebensqualität, d.h. Einschränkungen im Alltag und Schlafstörungen einen Trend zur Verbesserung.

Relevant für die Praxis ist besonders die Wirksamkeit des probiotischen für die Subgruppe Kinder, die aufgrund der hohen Prävalenz der atopischen Dermatitis in dieser Altersgruppe die Hauptanwender des Produktes darstellen. Auch hier konnten trotz der geringen Grußppengröße (n=7) statistisch signifikante Verbesserungen von SCORAD und local SCORAD erzielt werden.

Wie bereits erwähnt, müssen alle Ergebnisse der klinischen Analyse unter Berücksichtigung des Studiendesigns bewertet werden, welches ein Absetzen aller wirkstoffhaltigen Präparate sieben Tage vor dem Beginn der Testphase beinhaltete. Diese strikte Vorgabe wurde gewählt, um die beobachteten Effekte eindeutig auf den Einfluss des Testproduktes zurückführen zu können und alle Teilnehmer unter vergleichbaren Bedingungen zu betrachten.

Unter diesen Voraussetzungen bestand natürlich das Risiko, dass durch das Absetzen der wirkstoffhaltigen Präparate eine Verschlechterung der Symptomatik eintritt, welche durch die Wirkung des Bades nicht abgemildert werden kann. Das Vorgehen könnte weiterhin zu einer entsprechend kritischen Erwartungshaltung von Studienteilnehmern beigetragen haben, die der Wirkung ihrer bisherigen Therapie vertrauen, während sie dem unbekannten Testprodukt möglicherweise eher skeptisch gegenüberstanden.

Es ist daher bemerkenswert, dass im Mittel für die gesamte Studienpopulation sowie für beide Behandlungsgruppen eine Verbesserung Symptomatik dokumentiert werden konnte.

### 7.2 Mikrobiologische Untersuchung

Die Genkopienzahl von S. aureus sank in beiden Behandlungsgruppen. In der Behandlungsgruppe mit der niedrigeren Dosierung sowie der Gesamtpopulation waren diese Unterschiede zwischen Tag 0 und Tag 14 statistisch signifikant. Zugleich zeigte die Analyse der bakteriellen Mikrobiome eine tendenzielle Reduktion der relativen Abundanz der Gattung Staphylococcus, die jedoch nicht signifikant war. Da unterschiedliche PCR-Bedingungen und Primer für die qPCR (Primer für das nucA-Gen) bzw. für die AmpliconSequenzierung (Primer für das bakterielle 16S rRNA Gen) verwendet wurden, ist die Genkopienzahl aufgrund der höheren Spezifität des gewählten qPCR Ansatzes aussagekräftiger als die relative Abundanz, die durch die Amplicon-Hochdurchsatzsequenzierung generiert wurde. Trotzdem lässt sich das Ergebnis der Abnahme von Staphylococcen mit beiden voneinander unabhängigen PCR basierten Verfahren bestätigen. Die Ergebnisse der Ampliconsequenzierung lassen keine phylogenetische Auflösung unterhalb der Gattungsebene von Staphylococcus zu. Daher können Verschiebungen der Quantität von S. aureus zu anderen Spezies innerhalb der Gattung Staphylococcus, wie z.B. S. epidermidis, der auf gesunder Haut sehr häufig zu finden ist, nur theoretisch angenommen werden.

Die Konzentration von S. aureus auf der Haut wird schon seit langem als wesentlicher Faktor für den Schweregrad der atopischen Dermatitis interpretiert. Die Reduktion der Genkopienzahl von S. aureus steht daher im Einklang mit der Verbesserung der klinischen Symptomatik, die mittels SCORAD- und des Teilnehmer-Fragebogens erhoben wurde. Diese Korrelation lässt den Schluss zu, dass eine Reduktion von S. aureus - neben eventuell weiteren Parametern- dazu beigetragen haben kann, die klinische Verbesserung der Symptomatik zu erzielen.

Eine Besonderheit des Prüfpräparates im Vergleich zu anderen probiotischen dermatologischen Produkten stellt die Verwendung lebender Mikroorganismen dar. Diese finden sich schließlich in der mikrobiologischen Analyse der Hautabstriche wieder: Die Sequenzmotive von bakteriellen Bestandteilen des probiotischen Badezusatzes wurden in beiden Behandlungsgruppen über die Zeit immer häufiger in den bakteriellen Hautmikrobiomen der Teilnehmer detektiert. Dabei erhöhten sich die Anteile der Gattungen Lactobacillus und Bifidobacterium bereits innerhalb der ersten Behandlungstage und schienen sich danach auf diesem höheren Niveau zu stabilisieren. Dieses Ergebnis ist indikativ, dass die Bakterien des probiotischen Badezusatzes nicht nur die vorhandene Diversität beeinflussten und somit auf unterschiedlichen Ebenen (Kommensalismus, Konkurrenz, Mutualismus) in Interaktion mit dem vorhandenen Hautmikrobiom traten, sondern selbst -zumindest während des Behandlungszeitraumes- Teil des Hautmikrobioms wurden. Als Bestandteil des Hautmikrobioms können diese Bakterien eigenständig eine Interaktion mit den dermalen Hautzellen und dem Immunsystem des Wirtes eingehen und in der Quantität und Qualität einen höheren Anteil an Interaktionen mit dem vorhandenen Hautmikrobiom vollziehen. Da sich die relative Abundanz dieser Gattungen während des Anwendungszeitraum erhöhte, ist zu postulieren, dass diese Interaktionsebene mit dem Hautmikrobiom und den Hautepithelzellen quantitativ und zeitlich nachhaltig auf- und ausgebaut werden kann.

Insgesamt zeigt die Behandlung einen positiven Einfluss auf die bakterielle Richness (Diversität) und erhöhte auch die relative Abundanz von Mitgliedern des bakteriellen Mikrobioms, die nicht Bestandteil des probiotischen Badezusatzes waren. Es ist möglich, dass diese zusätzlichen Mitglieder des bakteriellen Hautmikrobioms zuvor von anderen Bestandteilen des Hautmikrobioms, insbesondere S. aureus unterdrückt und unter die Detektionsgrenze reduziert wurden. Weiterhin könnte auch die Behandlung mit dem probiotischen Badezusatz den Besiedelungserfolg der Mikroorganismen von anderen Hautpartien, der Kleidung, der Luft oder des Wassers erhöht haben. Da es keine Kontrollgruppe bzw. Placebogruppe gab, mit denen die gleichen Analysen zur gleichen Zeit durchgeführt worden sind, kann kein eindeutiger Grund für die Erhöhung der Reichhaltigkeit identifiziert werden. Es ist jedoch davon auszugehen, dass die mit der atopischen Dermatitis einhergehende Dysbiose gelindert wurde.

Bei einer gesunden Haut besteht eine synergistische Interaktion zwischen dem Hautmikrobiom und dem menschlichen Wirt, bei der die Immunantwort und die Abwehr von Krankheitserregern aufeinander abgestimmt sind. Umso mehr eine Dysbiose vorherrscht, umso weniger können diese Funktionen erfüllt werden. Die Behandlung mit dem probiotischen Badezusatz fördert also den Studienergebnissen zufolge die Regenerierung der Reichhaltigkeit und der Diversität des Hautmikrobioms und unterstützt so deren Funktion für die Wirtinteraktion.

Die Analyse der zeitlichen Entwicklung der Zusammensetzung des bakteriellen Hautmikrobioms zeigt, dass die Proben der Teilnehmer beider Behandlungsgruppen sich von der Ausgangszusammensetzung weg entwickelten. Die Probanden, die mit der niedrigeren Dosis behandelt wurden, wiesen tendenziell eine stabilere Zusammensetzung der bakteriellen Gemeinschaft auf als Probanden, die eine höhere Dosis angewendet hatten. Die Teilbäder haben demnach einen zeitlichen Effekt und ein Aufbau einer reichhaltigeren Diversität kann bereits innerhalb von sieben Behandlungstagen erfolgen. Da im Rahmen dieser Studie keine höhere zeitliche Auflösung der Veränderungen des Mikrobioms erhoben wurde, kann theoretisch auch eine kürzere Behandlungsdauer für diesen Effekt postuliert werden. Weitere Experimente und Analysen könnten den zeitlichen Verlauf höher auflösen.

Die Auswertung der Subgruppe der Kinder bestätigt die in der Gesamtpopulation beobachteten Trends in Bezug auf die mikrobiologischen Ergebnisse sowie bereits für die klinische Symptomatik. Es kann also auch von einer vergleichbaren Wirksamkeit bei Kindern ausgegangen werden, wenngleich die Ergebnisse aufgrund der geringen Teilnehmerzahlen in dieser Subgruppe statistisch nicht signifikant sind.

### 7.3 Fazit

Zusammenfassend lässt sich feststellen, dass die Anwendung des probiotischen Bades bei Probanden mit atopischer Dermatitis im Mittel eine Reduktion der Besiedlung der Haut mit S. aureus bewirkte und zugleich zu einer höheren Diversität des bakteriellen Hautmikrobioms führte. Diese Effekte konnten bereits zum ersten Untersuchungszeitpunkt nach siebentägiger Anwendung festgestellt werden und betreffen mit marginalen Unterschieden beide Untersuchungsgruppen, die mit jeweils unterschiedlichen Dosierungen des probiotischen Bades behandelt wurden. Aufgrund des Studiendesigns, welches eine Anwendung anderer wirkstoffhaltiger Präparate während des Studienzeitraumes sowie innerhalb einer vorausgehenden siebentägigen Auswaschphase ausschloss, können alle dokumentierten Veränderungen relativ eindeutig auf das Testprodukt zurückgeführt werden.

Die Ergebnisse der mikrobiologischen Untersuchungen stehen im Einklang mit der statistisch signifikanten Verbesserung der klinischen Symptomatik, die mithilfe des SCORADs durch die Studienärzte sowie mithilfe eines Teilnehmerfragebogens dokumentiert wurde.

Aufgrund der Korrelation der klinischen und der mikrobiologischen Ergebnisse kann postuliert werden, dass die klinische Wirkung des probiotischen Bades zumindest teilweise auf einer der Beeinflussung einer bestehenden dermalen Dysbiose beruht. Zugleich unterstreichen die Ergebnisse die Bedeutung des Hautmikrobioms für die Pathogenese der atopischen Dermatitis.

## Patentansprüche

1. Pharmazeutische Zusammensetzung zur Verwendung bei der Behandlung von Neurodermitis, umfassend ein Probiotikum,
wobei das Probiotikum die Mikroorganismen *Lactobacillus paracasei, Bifidobacterium longum, Streptococcus thermophilus, Lactobacillus johnsonii, Lactobacillus reuteri, Lactobacillus plantarum, Lactobacillus gasseri, Lactobacillus rhamnosus* und *Bifidobacterium lactis* umfasst.

2. Pharmazeutische Zusammensetzung zur Verwendung gemäß Anspruch 1,
wobei die Gesamtkonzentration der Mikroorganismen in der Zusammensetzung im Bereich von 0,2 x 10⁹ KbE/g bis 3,0 x 10⁹ KbE/g (KbE = koloniebildende Einheiten), optional im Bereich von 0,6 x 10⁹ KbE/g bis 2,4 x 10⁹ KbE/g, ferner optional im Bereich von 1,0 x 10⁹ KbE/g bis 2,0 x 10⁹ KbE/g liegt.

3. Pharmazeutische Zusammensetzung zur Verwendung gemäß Anspruch 2,
wobei die Gesamtkonzentration der Mikroorganismen in der Zusammensetzung ungefähr 1,8 x 10⁹ KbE/g ist.

4. Pharmazeutische Zusammensetzung zur Verwendung gemäß Anspruch 1,
wobei die Zusammensetzung ein Präbiotikum umfasst, welches vorzugsweise aus der Gruppe bestehend aus Inulin, Maltodextrin, Pektin und resistente Starke ausgewählt ist, besonders bevorzugt Inulin und/oder Maltodextrin ist.

5. Pharmazeutische Zusammensetzung zur Verwendung gemäß einem der vorstehenden Anspruche 1-4, die Zusammensetzung als topische Darreichungsform, insbesondere als Creme, Salbe oder Öl, oder als Badezusatz vorliegt.

6. Wasserbad, umfassend 0,5 g bis 10 g, optional 1,0 g bis 8,0 g, ferner optional 2,0 g bis 6,0 g, oder optional 2,5 g oder 5,0 g einer pharmazeutischen Zusammensetzung gemäß einem der Anspruche 1-4 pro Liter Wasser.

7. Verfahren zur Herstellung eines Wasserbades gemäß Anspruch 6, umfassend
Mischen der pharmazeutischen Zusammensetzung gemäß einem der Anspruche 1-4 mit Wasser, so dass das Bad eine Konzentration der Zusammensetzung von 0,5 g bis 10 g pro Liter Wasser aufweist.

## Claims

1. A pharmaceutical composition for use in the treatment of atopic dermatitis, comprising a probiotic,
wherein the probiotic comprises the microorganisms *Lactobacillus paracasei, Bifidobacterium longum, Streptococcus thermophilus, Lactobacillus johnsonii, Lactobacillus reuteri, Lactobacillus plantarum, Lactobacillus gasseri, Lactobacillus rhamnosus* and *Bifidobacterium lactis.*

2. A pharmaceutical composition for use according to claim 1,
wherein the total concentration of microorganisms in the composition ranges from 0.2 x 10⁹ CFU/g to 3.0 x 10⁹ CFU/g (CFU = colony-forming units), optionally from 0.6 x 10⁹ CFU/g to 2.4 x 10⁹ CFU/g, furthermore optionally from 1.0 x 10⁹ CFU/g to 2.0 x 10⁹ CFU/g.

3. A pharmaceutical composition for use according to claim 2,
wherein the total concentration of microorganisms in the composition is approximately 1.8 x 10⁹ CFU/g.

4. A pharmaceutical composition for use according to claim 1,
wherein the composition comprises a prebiotic which is preferably selected from the group consisting of inulin, maltodextrin, pectin and resistant starch, particularly preferably is inulin and/or maltodextrin.

5. A pharmaceutical composition for use according to any of the preceding claims 1-4, wherein the composition exists as a topical dosage form, in particular as a cream, ointment or oil, or as a bath additive.

6. A water bath comprising 0.5 g to 10 g, optionally 1.0 g to 8.0 g, furthermore optionally 2.0 g to 6.0 g, or optionally 2.5 g or 5.0 g of a pharmaceutical composition according to any of claims 1-4 per litre of water.

7. A method of producing a water bath according to claim 6, comprising
mixing the pharmaceutical composition according to any of claims 1-4 with water so that the bath has a concentration of the composition of 0.5 g to 10 g per litre of water.

## Revendications

1. Composition pharmaceutique à utiliser dans le traitement de la dermatite atopique, comprenant un probiotique,
le probiotique comprenant les micro-organismes *Lactobacillus paracasei, Bifidobacterium longum, Streptococcus thermophilus, Lactobacillus johnsonii, Lactobacillus reuteri, Lactobacillus plantarum, Lactobacillus gasseri, Lactobacillus rhamnosus* et *Bifidobacterium lactis.*

2. Composition pharmaceutique à utiliser selon la revendication 1,
dans laquelle la concentration totale de micro-organismes dans la composition est comprise entre 0,2 x 10⁹ UFC/g et 3,0 x 10⁹ UFC/g (UFC = unités formant colonies), éventuellement entre 0,6 x 10⁹ UFC/g et 2,4 x 10⁹ UFC/g, en outre éventuellement entre 1,0 x 10⁹ UFC/g et 2,0 x 10⁹ UFC/g.

3. Composition pharmaceutique à utiliser selon la revendication 2,
dans laquelle la concentration totale de micro-organismes dans la composition est d'environ 1,8 x 10⁹ UFC/g.

4. Composition pharmaceutique à utiliser selon la revendication 1,
dans laquelle la composition comprend un prébiotique qui, de préférence, est choisi dans le groupe constitué par l'inuline, la maltodextrine, la pectine et l'amidon résistant, plus préférablement est l'inuline et/ou la maltodextrine.

5. Composition pharmaceutique à utiliser selon l'une des revendications 1 à 4 précédentes, dans laquelle la composition existe sous forme de formulation topique, notamment de crème, d'onguent ou d'huile, ou comme additif de bain.

6. Bain-marie comprenant 0,5 g à 10 g, éventuellement 1,0 g à 8,0 g, en outre éventuellement 2,0 g à 6,0 g, ou éventuellement 2,5 g ou 5,0 g d'une composition pharmaceutique selon l'une des revendications 1 à 4 par litre d'eau.

7. Procédé de fabrication d'un bain-marie selon la revendication 6, comprenant
mélanger la composition pharmaceutique selon l'une des revendications 1 à 4 avec de l'eau de telle sorte que le bain ait une concentration de la composition de 0,5 g à 10 g par litre d'eau.
